(19) Europäisches Patentamt
European Patent Office
Office européen des brevets

(11) EP 2 604 277 A1

(12) EUROPEAN PATENT APPLICATION

(43) Date of publication:
**19.06.2013 Bulletin 2013/25**

(51) Int Cl.:
***A61K 39/395*** *(2006.01)* ***A61P 35/00*** *(2006.01)*

(21) Application number: **13155433.9**

(22) Date of filing: **13.10.2008**

(84) Designated Contracting States:
**AT BE BG CH CY CZ DE DK EE ES FI FR GB GR HR HU IE IS IT LI LT LU LV MC MT NL NO PL PT RO SE SI SK TR**

(30) Priority: **15.10.2007 EP 07020120**

(62) Document number(s) of the earlier application(s) in accordance with Art. 76 EPC:
**08839967.0 / 2 203 185**

(27) Previously filed application:
**13.10.2008 PCT/EP2008/008635**

(71) Applicant: **Roche Glycart AG**
**8952 Schlieren (CH)**

(72) Inventors:
- **Braemer, Pamela**
**69469 Weinheim (DE)**
- **Friess, Thomas**
**86911 Diessen-Dettenhofen (DE)**
- **Klein, Christian**
**8906 Bonstetten (CH)**
- **Umana, Pablo**
**8832 Wollerau (CH)**

(74) Representative: **Burger, Alexander et al**
**Roche Diagnostics GmbH**
**Patent Department (LPP.....6164)**
**P.O.Box 11 52**
**82372 Penzberg (DE)**

Remarks:
This application was filed on 15-02-2013 as a divisional application to the application mentioned under INID code 62.

# (54) Combination therapy of a type II anti-CD20 antibody with an anti-Bcl-2 active agent

(57) The present invention is directed to the use of a type II anti-CD20 antibody for the manufacture of a medicament for the treatment of cancer, especially of CD20 expressing cancers in combination with an anti-Bcl-2 active agent.

EP 2 604 277 A1

## Description

The present invention is directed to the use a type II anti-CD20 antibody for the manufacture of a medicament for the treatment of cancer, especially of CD20 expressing cancers in combination with an anti-Bcl-2 active agent.

Background of the Invention

The CD20 molecule (also called human B-lymphocyte-restricted differentiation antigen or Bp35) is a hydrophobic transmembrane protein with a molecular weight of approximately 35 kD located on pre-B and mature B lymphocytes (Valentine, M.A., et al., J. Biol. Chem. 264 (19) (1989) 11282-11287; and Einfield, D.A., et al. EMBO J. 7(3) (1988) 711-717). CD20 is found on the surface of greater than 90% of B cells from peripheral blood or lymphoid organs and is expressed during early pre-B cell development and remains until plasma cell differentiation. CD20 is present on both normal B cells as well as malignant B cells. In particular, CD20 is expressed on greater than 90% of B cell non-Hodgkin's lymphomas (NHL) (Anderson, K.C., et al., Blood 63(6) (1984) 1424-1433) but is not found on hematopoietic stem cells, pro-B cells, normal plasma cells, or other normal tissues (Tedder, T.F., et al., J, Immunol. 135(2) (1985) 973-979).

The 85 amino acid carboxyl-terminal region of the CD20 protein is located within the cytoplasm. The length of this region contrasts with that of other B cell-specific surface structures such as IgM, IgD, and IgG heavy chains or histocompatibility antigens class I1 a or β chains, which have relatively short intracytoplasmic regions of 3, 3, 28, 15, and 16 amino acids, respectively (Komaromy, M., et al., NAR 11 (1983) 6775-6785). Of the last 61 carboxyl-terminal amino acids, 21 are acidic residues, whereas only 2 are basic, indicating that this region has a strong net negative charge. The GenBank Accession No. is NP-690605. It is thought that CD20 might be involved in regulating an early step (s) in the activation and differentiation process of B cells (Tedder, T.F., et al., Eur. J. Immunol. 16 (1986) 881-887) and could function as a calcium ion channel (Tedder, T.F., et al., J. Cell. Biochem. 14D (1990) 195).

There exist two different types of anti-CD20 antibodies differing significantly in their mode of CD20 binding and biological activities (Cragg, M.S., et al., Blood 103 (2004) 2738-2743; and Cragg, M.S., et al., Blood 101 (2003) 1045-1052). Type I antibodies, as e.g. rituximab, are potent in complement mediated cytotoxicity, whereas type II antibodies, as e.g. Tositumomab (B1), 11B8, AT80 or humanized B-Ly1 antibodies, effectively initiate target cell death via caspase-independent apoptosis with concomitant phosphatidylserine exposure.

The sharing common features of type I and type II anti-CD20 antibodies are summarized in Table 1.

Table 1:

| Properties of type I and type II anti-CD20 antibodies | |
|---|---|
| type I anti-CD20 antibodies | type II anti-CD20 antibodies |
| type I CD20 epitope | type II CD20 epitope |
| Localize CD20 to lipid rafts | Do not localize CD20 to lipid rafts |
| Increased CDC (if IgG1 isotype) | Decreased CDC (if IgG1 isotype) |
| ADCC activity (if IgG1 isotype) | ADCC activity (if IgG1 isotype) |
| Full binding capacity | Reduced binding capacity |
| Homotypic aggregation | Stronger homotypic aggregation |
| Apoptosis induction upon cross-linking | Strong cell death induction without cross-linking |

The Bcl-2 family of proteins regulates programmed cell death triggered by developmental cues and in response to multiple Stress signals (Cory. S., and Adams, J.M., Nature Reviews Cancer 2 (2002) 647-656; Adams, Genes und Development 17 (2003) 2481-2495; Danial, N.N., and Korsmeyer, S.J., Cell 116 (2004) 205-219). Whereas cell survival is promoted by Bcl-2 itself and several close relatives (Bcl-xL, Bcl-W, Mcl-1 and Al), which bear three or four conserved Bcl-2 homology (BH) regions, apoptosis is driven by two other sub-families. The initial signal for cell death is conveyed by the diverse group of BH3-only proteins, including Bad, Bid, Bim, Puma and Noxa, which have in common only the small BH3 interaction domain (Huang and Strasser, Ce11 103 (2000) 839-842). However, Bax or Bak, multi-domain proteins containing BH1-BH3, are required for commitment to cell death (Cheng, et al., Molecular Cell 8 (2001) 705-711; Wei, M.C., et al., Science 292 (2001) 727-730; Zong, W.X., et al., Genes and Development 15 148 (2001) 1-1486). When activated, they can permeabilize the outer membrane of mitochondria and release pro-apoptogenic factors (e.g. cytochrome C) needed to activate the caspases that dismantle the cell (Wang, K., Genes and Development 15 (2001) 2922-2933; (Adams, 2003 supra); Green, D.R., and Kroemer, G., Science 305 (2004) 626-629).

Interactions between members of these three factions of the Bcl-2 family dictate whether a cell lives or dies. When BH3-only proteins have been activated, for example, in response to DNA damage, they can bind via their BH3 domain to a groove on their pro-survival relatives (Sattler, et al., Science 275 (1997) 983-986). How the BH3-only and Bcl-2-like proteins control the activation of Bax and Bak, however, remains poorly understood (Adams, 2003 supra). Most attention has focused on Bax. This soluble monomeric protein (Hsu, Y.T., et al., Journal of Biological Chemistry 272 (1997) 13289-1 3834; Wolter, K.G., et al., Journal of Cell Biology 139 (1997) 1281-92) normally has its membrane targeting domain inserted into its groove, probably accounting for its cytosolic localization (Nechushtan, A., et al., EMBO Journal 18 (1999) 2330- 2341; Suzuki, et al., Cell 103 (2000) 645-654; Schinzel, A., et al., J Cell Biol 164 (2004) 1021-1032). Several unrelated peptides/proteins have been proposed to modulate Bax activity reviewed in (Lucken-Ardjomande, S., and Martinou, J.C., J Cell Sci 118 (2005) 473-483), but their physiological relevance remains to be established. Alternatively, Bax may be activated via direct engagement by certain BH3-only proteins (Lucken-Ardjomande, S., and Martinou, J.C, 2005 supra), the best documented being a truncated form of Bid, tBid (Wei, M.C., et al., Genes und Development 14 (2000) 2060-2071; Kuwana, T., et al., Cell 111 (2002) 331-342; Roucou, X., et al., Biochemical Journal 368 (2002) 915-921; Cartron, P.F., et al., Mol Cell 16 (2004) 807-818). As discussed elsewhere (Adams 2003 supra), the oldest model, in which Bcl-2 directly engages Bax (Oltvai, Z.N., et al., Cell 74 (1993) 609-619), has become problematic because Bcl-2 is membrane bound while Bax is cytosolic, and their interaction seems highly dependent on the detergents used for cell lysis (Hsu, Y.T., and Youle, 1997 supra). Nevertheless, it is well established that the BH3 region of Bax can mediate association with Bcl-2 (Zha, H., and Reed, J., Journal of Biological Chemistry 272 (1997) 31482-88; Wang, K., et al., Molecular und Cellular Biology 18 (1998) 6083-6089) and that Bcl-2 prevents the oligomerization of Bax, even though no heterodimers can be detected (Mikhailov, V., et al., Journal of Biological Chemistry 276 (2001) 18361-18374). Thus, whether the pro-survival proteins restrain Bax activation directly or indirectly remains uncertain.

Although Bax and Bak seem in most circumstances to be functionally equivalent (Lindsten, T., et al., Molecular Cell 6 (2000) 1389-1399; Wei, M.C., et al., 2001 supra), substantial differences in their regulation would be expected from their distinct localization in healthy cells. Unlike Bax, which is largely cytosolic, Bak resides in complexes on the outer membrane of mitochondria and on the endoplasmic reticulum of healthy cells (Wei, M.C., et al., 2000 supra; Zong, W.X., et al., Journal of Ce11 Biology 162 (2003) 59-69). Nevertheless, on receipt of cytotoxic signals, both Bax and Bak change conformation, and Bax translocates to the organellar membranes, where both Bax and Bak then form homo-oligomers that can associate, leading to membrane permeabilization (Hsu, Y.T., et al., PNAS 94 (1997) 3668-3672; Wolter, K.G., et al., 1997 supra; Antonsson, B., et al., Journal of Biological Chemistry 276 (2001) 11615-11623; Nechushtan, A., et al., Journal of Cell Biology 153 (2001) 1265-1276; Wei, M.C., et al., 2001 supra; Mikhailov, V., et al., Journal of Biological Chemistry 278 (2003) 5367-5376).

There exist various Bcl-2 inhibitors, which all have the same property of inhibiting prosurvival members of the Bcl-2 family of proteins and are therefore promising candidates for the treatment of cancer. Such Bcl-2 inhibitors are e.g. Oblimersen, SPC-2996, RTA-402, Gossypol, AT-101, Obatoclax mesylate, A-371191, A-385358, A-438744, ABT-737, AT-101, BL-11, BL-193, GX-15-003, 2-Methoxyantimycin $A_3$, HA-14-1, KF-67544, Purpurogallin, TP-TW-37, YC-137 and Z-24, and are described e.g. in Zhai, D., et al., Cell Death and Differentiation 13 (2006) 1419-1421.

Smith, M. R., et al, Molecular Cancer Therapeutics 3(12) (2004) 1693-1699 and Ramanarayanan, J. et al., British Journal of Haematology 127(5) (2004) 519-530, refer to a combination of , a type I anti-CD20 antibody (rituximab) with antisense Bcl-2 oligonucleotides (Oblimersen).

## Summary of the Invention

The invention comprises the use of a type II anti-CD20 antibody for the manufacture of a medicament for the treatment of CD20 expressing cancer in combination with an anti-Bcl-2 active agent.

The invention further comprises the use of a type II anti-CD20 antibody for the manufacture of a medicament for the treatment of a patient suffering from a CD20 expressing cancer in combination with an anti-Bcl-2 active agent.

Preferably said anti-Bcl-2 active agent is a Bcl-2 inhibitor with an IC50 of the anti-Bcl-2 inhibitory activity of 5 $\mu$M or less.

Preferably said type II anti-CD20 antibody has a ratio of the binding capacities to CD20 on Raji cells (ATCC-No. CCL-86) of said type II anti-CD20 antibody compared to rituximab of 0.3 to 0.6, more preferably 0.35 to 0.55, and still more preferably 0.4 to 0.5

Preferably said type II anti-CD20 antibody is a humanized B-Ly1 antibody.

Preferably said type II anti-CD20 antibody has increased antibody dependent cellular cytotoxicity (ADCC).

Preferably said anti-Bcl-2 active agent is selected from the group consisting of Oblimersen, SPC-2996, RTA-402, Gossypol, AT-101, Obatoclax mesylate, A-371191, A-385358, A-438744, ABT-737, AT-101, BL-11, BL-193, GX-15-003, 2-Methoxyantimycin $A_3$, HA-14-1, KF-67544, Purpurogallin, TP-TW-37, YC-137 and Z-24, more preferably from the group consisting of ABT-263and ABT-737.Preferably the CD20 expressing cancer is a B-Cell Non-Hodgkin's lymphoma (NHL).

Detailed Description of the Invention

The term "antibody" encompasses the various forms of antibodies including but not being limited to whole antibodies, human antibodies, humanized antibodies and genetically engineered antibodies like monoclonal antibodies, chimeric antibodies or recombinant antibodies as well as fragments of such antibodies as long as the characteristic properties according to the invention are retained.

The terms "monoclonal antibody" or "monoclonal antibody composition" as used herein refer to a preparation of antibody molecules of a single amino acid composition. Accordingly, the term "human monoclonal antibody" refers to antibodies displaying a single binding specificity which have variable and constant regions derived from human germline immunoglobulin sequences. In one embodiment, the human monoclonal antibodies are produced by a hybridoma which includes a B cell obtained from a transgenic non-human animal, e.g. a transgenic mouse, having a genome comprising a human heavy chain transgene and a light human chain transgene fused to an immortalized cell.

Preferably said type II anti-CD20 antibody is a monoclonal antibody.

The term "chimeric antibody" refers to a monoclonal antibody comprising a variable region, i.e., binding region, from one source or species and at least a portion of a constant region derived from a different source or species, usually prepared by recombinant DNA techniques. Chimeric antibodies comprising a murine variable region and a human constant region are especially preferred. Such murine/human chimeric antibodies are the product of expressed immunoglobulin genes comprising DNA segments encoding murine immunoglobulin variable regions and DNA segments encoding human immunoglobulin constant regions. Other forms of "chimeric antibodies" encompassed by the present invention are those in which the class or subclass has been modified or changed from that of the original antibody. Such "chimeric" antibodies are also referred to as "class-switched antibodies." Methods for producing chimeric antibodies involve conventional recombinant DNA and gene transfection techniques now well known in the art. See, e.g., Morrison, S.L., et al., Proc. Natl. Acad Sci. USA 81 (1984) 6851-6855; US 5,202,238 and US 5,204,244.

The term "humanized antibody" refers to antibodies in which the framework or "complementarity determining regions" (CDR) have been modified to comprise the CDR of an immunoglobulin of different specificity as compared to that of the parent immunoglobulin. In a preferred embodiment, a murine CDR is grafted into the framework region of a human antibody to prepare the "humanized antibody." See, e.g., Riechmann, L., et al., Nature 332 (1988) 323-327; and Neuberger, M.S., et al., Nature 314 (1985) 268-270. Particularly preferred CDRs correspond to those representing sequences recognizing the antigens noted above for chimeric and bifunctional antibodies.

The term "human antibody", as used herein, is intended to include antibodies having variable and constant regions derived from human germline immunoglobulin sequences. Human antibodies are well-known in the state of the art (van Dijk, M.A., and van de Winkel, J.G., Curr. Opin. Pharmacol. 5 (2001) 368-374). Based on such technology, human antibodies against a great variety of targets can be produced. Examples of human antibodies are for example described in Kellermann, S. A., et al., Curr Opin Biotechnol. 13 (2002) 593-597.

The term "recombinant human antibody", as used herein, is intended to include all human antibodies that are prepared, expressed, created or isolated by recombinant means, such as antibodies isolated from a host cell such as a NS0 or CHO cell or from an animal (e.g. a mouse) that is transgenic for human immunoglobulin genes or antibodies expressed using a recombinant expression vector transfected into a host cell. Such recombinant human antibodies have variable and constant regions derived from human germline immunoglobulin sequences in a rearranged form. The recombinant human antibodies according to the invention have been subjected to in vivo somatic hypermutation. Thus, the amino acid sequences of the VH and VL regions of the recombinant antibodies are sequences that, while derived from and related to human germline VH and VL sequences, may not naturally exist within the human antibody germline repertoire in vivo.

As used herein, "specifically binding" or "binds specifically to" refers to an antibody specifically binding to the CD20 antigen. Preferably the binding affinity is of KD-value of $10^{-9}$ mol/l or lower (e.g. $10^{-10}$ mol/l), preferably with a KD-value of $10^{-10}$ mol/l or lower (e.g. $10^{-12}$ mol/l). The binding affinity is determined with a standard binding assay, such as Scatchard plot analysis on CD20 expressing cells.

The term "nucleic acid molecule", as used herein, is intended to include DNA molecules and RNA molecules. A nucleic acid molecule may be single-stranded or double-stranded, but preferably is double-stranded DNA.

The "constant domains" are not involved directly in binding the antibody to an antigen but are involved in the effector functions (ADCC, complement binding, and CDC).

The "variable region" (variable region of a light chain (VL), variable region of a heavy chain (VH)) as used herein denotes each of the pair of light and heavy chains which is involved directly in binding the antibody to the antigen. The domains of variable human light and heavy chains have the same general structure and each domain comprises four framework (FR) regions whose sequences are widely conserved, connected by three "hypervariable regions" (or complementarity determining regions, CDRs). The framework regions adopt a b-sheet conformation and the CDRs may form

loops connecting the b-sheet structure. The CDRs in each chain are held in their three-dimensional structure by the framework regions and form together with the CDRs from the other chain the antigen binding site. The antibody heavy and light chain CDR3 regions play a particularly important role in the binding specificity/affinity of the antibodies according to the invention and therefore provide a further object of the invention.

The terms "hypervariable region" or "antigen-binding portion of an antibody" when used herein refer to the amino acid residues of an antibody which are responsible for antigen-binding. The hypervariable region comprises amino acid residues from the "complementarity determining regions" or "CDRs". "Framework" or "FR" regions are those variable domain regions other than the hypervariable region residues as herein defined. Therefore, the light and heavy chains of an antibody comprise from N- to C-terminus the domains FR1, CDR1, FR2, CDR2, FR3, CDR3, and FR4. Especially, CDR3 of the heavy chain is the region which contributes most to antigen binding. CDR and FR regions are determined according to the standard definition of Kabat, et al., Sequences of Proteins of Immunological Interest, 5th Ed. Public Health Service, National Institutes of Health, Bethesda, MD. (1991) and/or those residues from a "hypervariable loop".

The terms "CD20" and "CD20 antigen" are used interchangeably herein, and include any variants, isoforms and species homologs of human CD20 which are naturally expressed by cells or are expressed on cells transfected with the CD20 gene. Binding of an antibody of the invention to the CD20 antigen mediate the killing of cells expressing CD20 (e.g., a tumor cell) by inactivating CD20. The killing of the cells expressing CD20 may occur by one or more of the following mechanisms: Cell death/apoptosis induction, ADCC and CDC.

Synonyms of CD20, as recognized in the art, include B-lymphocyte antigen CD20, B-lymphocyte surface antigen B1, Leu-16, Bp35, BM5, and LF5.

The term "anti-CD20 antibody" according to the invention is an antibody that binds specifically to CD20 antigen. Depending on binding properties and biological activities of anti-CD20 antibodies to the CD20 antigen, two types of anti-CD20 antibodies (type I and type II anti-CD20 antibodies) can be distinguished according to Cragg, M.S., et al., Blood 103 (2004) 2738-2743; and Cragg, M.S., et al., Blood 101 (2003) 1045-1052, see Table 2.

Table 2:

| Properties of type I and type II anti-CD20 antibodies | |
|---|---|
| type I anti-CD20 antibodies | type II anti-CD20 antibodies |
| type I CD20 epitope | type II CD20 epitope |
| Localize CD20 to lipid rafts | Do not localize CD20 to lipid rafts |
| Increased CDC (if IgG1 isotype) | Decreased CDC (if IgG1 isotype) |
| ADCC activity (if IgG1 isotype) | ADCC activity (if IgG1 isotype) |
| Full binding capacity | Reduced binding capacity |
| Homotypic aggregation | Stronger homotypic aggregation |
| Apoptosis induction upon cross-linking | Strong cell death induction without cross-linking |

One essential property of type I and type II anti-CD20 antibody is their mode of binding. Thus, type I and type II anti-CD20 antibody can be classified by the ratio of the binding capacities to CD20 on Raji cells (ATCC-No. CCL-86) of said anti-CD20 antibody compared to rituximab.

The type II anti-CD20 antibodies have a ratio of the binding capacities to CD20 on Raji cells (ATCC-No. CCL-86) of said anti-CD20 antibody compared to rituximab of 0.3 to 0.6, preferably of 0.35 to 0.55, more preferably 0.4 to 0.5. Examples of such type II anti-CD20 antibodies include e.g. tositumomab (B1 IgG2a), humanized B-Ly1 antibody IgG1 (a chimeric humanized IgG1 antibody as disclosed in WO 2005/044859), 11B8 IgG1 (as disclosed in WO 2004/035607), and AT80 IgG1. Preferably said type II anti-CD20 antibody is a monoclonal antibody that binds to the same epitope as humanized B-Ly1 antibody (as disclosed in WO 2005/044859).

Type I anti-CD20 antibodies in contrast to the type II antibodies have a ratio of the binding capacities to CD20 on Raji cells (ATCC-No. CCL-86) of said anti-CD20 antibody compared to rituximab of 0.8 to 1.2, preferably of 0.9 to 1.1. Examples of such type I anti-CD20 antibodies include e.g. rituximab, 1F5 IgG2a (ECACC, hybridoma; Press, et al., Blood 69/2 (1987) 584-591), HI47 IgG3 (ECACC, hybridoma), 2C6 IgG1 (as disclosed in WO 2005/103081), 2F2 IgG1 (as disclosed and WO 2004/035607 and WO 2005/103081) and 2H7 IgG1 (as disclosed in WO 2004/056312).

The "ratio of the binding capacities to CD20 on Raji cells (ATCC-No. CCL-86) of an anti-CD20 antibodies compared to rituximab" is determined by direct immunofluorescence measurement (the mean fluorescence intensities (MFI) is measured) using said anti-CD20 antibody conjugated with Cy5 and rituximab conjugated with Cy5 in a FACSArray (Becton Dickinson) with Raji cells (ATCC-No. CCL-86), as described in Example No. 2, and calculated as follows:

$$\text{Ratio of the binding capacities to CD20 on Raji cells (ATCC-No. CCL-86)} = \frac{\text{MFI(Cy5-anti-CD20 antibody)}}{\text{MFI(Cy5-rituximab)}} \times \frac{\text{Cy5-labeling ratio(Cy5-rituximab)}}{\text{Cy5-labeling ratio(Cy5-anti-CD20 antibody)}}$$

MFI is the mean fluorescent intensity. The "Cy5-labeling ratio" as used herein means the number of Cy5-label molecules per molecule antibody.

Typically said type II anti-CD20 antibody has a ratio of the binding capacities to CD20 on Raji cells (ATCC-No. CCL-86) of said second anti-CD20 antibody compared to rituximab of 0.3 to 0.6, preferably 0.35 to 0.55, more preferably 0.4 to 0.5.

In a preferred embodiment said type II anti-CD20 antibody, preferably a humanized B-Ly1 antibody, has increased antibody dependent cellular cytotoxicity (ADCC).

By "antibody having increased antibody dependent cellular cytotoxicity (ADCC)" is meant an antibody, as that term is defined herein, having increased ADCC as determined by any suitable method known to those of ordinary skill in the art. One accepted in vitro ADCC assay is as follows:

1) the assay uses target cells that are known to express the target antigen recognized by the antigen-binding region of the antibody;

2) the assay uses human peripheral blood mononuclear cells (PBMCs), isolated from blood of a randomly chosen healthy donor, as effector cells;

3) the assay is carried out according to following protocol:

   i) the PBMCs are isolated using standard density centrifugation procedures and are suspended at 5 x $10^6$ cells/ml in RPMI cell culture medium;

   ii) the target cells are grown by standard tissue culture methods, harvested from the exponential growth phase with a viability higher than 90%, washed in RPMI cell culture medium, labeled with 100 micro-Curies of $^{51}Cr$, washed twice with cell culture medium, and resuspended in cell culture medium at a density of $10^5$ cells/ml;

   iii) 100 microliters of the final target cell suspension above are transferred to each well of a 96-well microtiter plate;

   iv) the antibody is serially-diluted from 4000 ng/ml to 0.04 ng/ml in cell culture medium and 50 microliters of the resulting antibody solutions are added to the target cells in the 96-well microtiter plate, testing in triplicate various antibody concentrations covering the whole concentration range above;

   v) for the maximum release (MR) controls, 3 additional wells in the plate containing the labeled target cells, receive 50 microliters of a 2% (VN) aqueous solution of non-ionic detergent (Nonidet, Sigma, St. Louis), instead of the antibody solution (point iv above);

   vi) for the spontaneous release (SR) controls, 3 additional wells in the plate containing the labeled target cells, receive 50 microliters of RPMI cell culture medium instead of the antibody solution (point iv above);

   vii) the 96-well microtiter plate is then centrifuged at 50 x g for 1 minute and incubated for 1 hour at 4°C;

   viii) 50 microliters of the PBMC suspension (point i above) are added to each well to yield an effector:target cell ratio of 25:1 and the plates are placed in an incubator under 5% CO2 atmosphere at 37°C for 4 hours;

   ix) the cell-free supernatant from each well is harvested and the experimentally released radioactivity (ER) is quantified using a gamma counter;

   x) the percentage of specific lysis is calculated for each antibody concentration according to the formula (ER-MR) /(MR-SR) x 100, where ER is the average radioactivity quantified (see point ix above) for that antibody concentration, MR is the average radioactivity quantified (see point ix above) for the MR controls (see point V above), and SR is the average radioactivity quantified (see point ix above) for the SR controls (see point vi above);

4) "increased ADCC" is defined as either an increase in the maximum percentage of specific lysis observed within the antibody concentration range tested above, and/or a reduction in the concentration of antibody required to achieve one half of the maximum percentage of specific lysis observed within the antibody concentration range tested above. The increase in ADCC is relative to the ADCC, measured with the above assay, mediated by the same antibody, produced by the same type of host cells, using the same standard production, purification, formulation and storage methods, which are known to those skilled in the art, but that has not been produced by host cells engineered to overexpress GnTIII.

Said "increased ADCC" can be obtained by glycoengineering of said antibodies, that means enhance said natural, cell-mediated effector functions of monoclonal antibodies by engineering their oligosaccharide component as described in Umana, P., et al., Nature Biotechnol. 17 (1999) 176-180 and US 6,602,684.

The term "complement-dependent cytotoxicity (CDC)" refers to lysis of human tumor target cells by the antibody according to the invention in the presence of complement. CDC is measured preferably by the treatment of a preparation of CD20 expressing cells with an anti-CD20 antibody according to the invention in the presence of complement. CDC is found if the antibody induces at a concentration of 100 nM the lysis (cell death) of 20% or more of the tumor cells after 4 hours. The assay is performed preferably with $^{51}Cr$ or Eu labeled tumor cells and measurement of released $^{51}Cr$ or Eu. Controls include the incubation of the tumor target cells with complement but without the antibody.

Typically type II anti-CD20 antibodies of the IgG1 isotype show characteristic CDC properties. Type II anti-CD20 antibodies have a decreased CDC (if IgG1 isotype) compared to type I antibodies of the IgG1 isotype. Preferably type II anti-CD20 antibodies are IgG1 isotype antibodies.

The "rituximab" antibody (reference antibody; example of a type I anti-CD20 antibody) is a genetically engineered chimeric human gamma 1 murine constant domain containing monoclonal antibody directed against the human CD20 antigen. This chimeric antibody contains human gamma 1 constant domains and is identified by the name "C2B8" in US 5,736,137 (Andersen, K.C., et. al.) issued on April 17,1998, assigned to IDEC Pharmaceuticals Corporation. Rituximab is approved for the treatment of patients with relapsed or refracting low-grade or follicular, CD20 positive, B cell non-Hodgkin's lymphoma. In vitro mechanism of action studies have shown that rituximab exhibits human complement--dependent cytotoxicity (CDC) (Reff, et. al., Blood 83(2) (1994) 435-445). Additionally, it exhibits significant activity in assays that measure antibody-dependent cellular cytotoxicity (ADCC).

The term "humanized B-Ly1 antibody" refers to humanized B-Ly1 antibody as disclosed in WO 2005/044859 and WO 2007/031875, which were obtained from the murine monoclonal anti-CD20 antibody B-Ly1 (variable region of the murine heavy chain (VH): SEQ ID NO: 1; variable region of the murine light chain (VL): SEQ ID NO: 2- see Poppema, S. and Visser, L., Biotest Bulletin 3 (1987) 131-139;) by chimerization with a human constant domain from IgG1 and following humanization (see WO 2005/044859 and WO 2007/031875). These "humanized B-Ly1 antibodies" are disclosed in detail in WO 2005/044859 and WO 2007/031875.

Preferably the "humanized B-Ly1 antibody" has variable region of the heavy chain (VH) selected from group of SEQ ID No.3 to SEQ ID No.20 (B-HH2 to B-HH9 and B-HL8 to B-HL17 of WO 2005/044859 and WO 2007/031875). Especially preferred are Seq. ID No. 3, 4, 7, 9, 11, 13 and 15 (B-HH2, BHH-3, B-HH6, B-HH8, B-HL8, B-HL11 and B-HL13 of WO 2005/044859 and WO 2007/031875). Preferably the "humanized B-Ly1 antibody" has variable region of the light chain (VL) of SEQ ID No. 20 (B-KV1 of WO 2005/044859 and WO 2007/031875). Furthermore the humanized B-Ly1 antibody is preferably an IgG1 antibody. Preferably such humanized B-Ly1 antibodies are glycoengineered (GE) in the Fc region according to the procedures described in WO 2005/044859, WO 2004/065540, WO 2007/031875, Umana, P., et al., Nature Biotechnol. 17 (1999) 176-180 and WO 99/154342. Such glycoengineered humanized B-Ly1 antibodies have an altered pattern of glycosylation in the Fc region, preferably having a reduced level of fucose residues. Preferably at least 40% or more (in one embodiment between 40% and 60%, in another embodiment at least 50%, and in still another embodiment at least 70% or more) of the oligosaccharides of the Fc region are non-fucosylated. Furthermore the oligosaccharides of the Fc region are preferably bisected.

The oligosaccharide component can significantly affect properties relevant to the efficacy of a therapeutic glycoprotein, including physical stability, resistance to protease attack, interactions with the immune system, pharmacokinetics, and specific biological activity. Such properties may depend not only on the presence or absence, but also on the specific structures, of oligosaccharides. Some generalizations between oligosaccharide structure and glycoprotein function can be made. For example, certain oligosaccharide structures mediate rapid clearance of the glycoprotein from the bloodstream through interactions with specific carbohydrate binding proteins, while others can be bound by antibodies and trigger undesired immune reactions. (Jenkins, N., et al., Nature Biotechnol. 14 (1996) 975-981).

Mammalian cells are the preferred hosts for production of therapeutic glycoproteins, due to their capability to glycosylate proteins in the most compatible form for human application. (Cumming, D.A., et al., Glycobiology 1 (1991) 115-130; Jenkins, N., et al., Nature Biotechnol. 14 (1996) 975-981). Bacteria very rarely glycosylate proteins, and like other types of common hosts, such as yeasts, filamentous fungi, insect and plant cells, yield glycosylation patterns associated with rapid clearance from the blood stream, undesirable immune interactions, and in some specific cases,

reduced biological activity. Among mammalian cells, Chinese hamster ovary (CHO) cells have been most commonly used during the last two decades. In addition to giving suitable glycosylation patterns, these cells allow consistent generation of genetically stable, highly productive clonal cell lines. They can be cultured to high densities in simple bioreactors using serum free media, and permit the development of safe and reproducible bioprocesses. Other commonly used animal cells include baby hamster kidney (BHK) cells, NSO- and SP2/0-mouse myeloma cells. More recently, production from transgenic animals has also been tested. (Jenkins, N., et al., Nature Biotechnol. 14 (1996) 975-981).

All antibodies contain carbohydrate structures at conserved positions in the heavy chain constant regions, with each isotype possessing a distinct array of N-linked carbohydrate structures, which variably affect protein assembly, secretion or functional activity. (Wright, A., and Monison, S. L., Trends Biotech. 15 (1997) 26-32). The structure of the attached N-linked carbohydrate varies considerably, depending on the degree of processing, and can include high-mannose, multiply-branched as well as biantennary complex oligosaccharides. (Wright, A., and Morrison, S. L., Trends Biotech. 15 (1997) 26-32). Typically, there is heterogeneous processing of the core oligosaccharide structures attached at a particular glycosylation site such that even monoclonal antibodies exist as multiple glycoforms. Likewise, it has been shown that major differences in antibody glycosylation occur between cell lines, and even minor differences are seen for a given cell line grown under different culture conditions. (Lifely, M. R., et al., Glycobiology 5 (1995) 813-822).

One way to obtain large increases in potency, while maintaining a simple production process and potentially avoiding significant, undesirable side effects, is to enhance the natural, cell-mediated effector functions of monoclonal antibodies by engineering their oligosaccharide component as described in Umana, P., et al., Nature Biotechnol. 17 (1999) 176-180 and US 6,602,684. IgG1 type antibodies, the most commonly used antibodies in cancer immunotherapy, are glycoproteins that have a conserved N-linked glycosylation site at Asn297 in each CH2 domain. The two complex biantennary oligosaccharides attached to Asn297 are buried between the CH2 domains, forming extensive contacts with the polypeptide backbone, and their presence is essential for the antibody to mediate effector functions such as antibody dependent cellular cytotoxicity (ADCC) (Lifely, M. R., et al., Glycobiology 5 (1995) 813-822; Jefferis, R., et al., Immunol. Rev. 163 (1998) 59-76; Wright, A., and Morrison, S. L., Trends Biotechnol. 15 (1997) 26-32).

It was previously shown that overexpression in Chinese hamster ovary (CHO) cells of ß(1,4)-N-acetylglucosaminyltransferase I11 ("GnTII17y), a glycosyltransferase catalyzing the formation of bisected oligosaccharides, significantly increases the in vitro ADCC activity of an antineuroblastoma chimeric monoclonal antibody (chCE7) produced by the engineered CHO cells. (See Umana, P., et al., Nature Biotechnol. 17 (1999) 176-180; and WO 99/154342, the entire contents of which are hereby incorporated by reference). The antibody chCE7 belongs to a large class of unconjugated monoclonal antibodies which have high tumor affinity and specificity, but have too little potency to be clinically useful when produced in standard industrial cell lines lacking the GnTIII enzyme (Umana, P., et al., Nature Biotechnol. 17 (1999) 176-180). That study was the first to show that large increases of ADCC activity could be obtained by engineering the antibody producing cells to express GnTIII, which also led to an increase in the proportion of constant region (Fc)-associated, bisected oligosaccharides, including bisected, non-fucosylated oligosaccharides, above the levels found in naturally-occurring antibodies.

The term "Bcl-2" as used herein refers to the Bcl-2 protein(Swiss Prot ID No. P10415), a member of the Bcl-2 family of proteins (Cory, S., and Adams, J.M., Nature Reviews Cancer 2 (2002) 647-656; Adams, Genes und Development 17 (2003) 2481-2495; Danial, N.N., and Korsmeyer, S.J., Cell 116 (2004) 205-219; Petros, A. M., Biochim Biophys Acta 1644 (2004) 83-94).

The term "anti-Bcl-2 active agent" comprises "anti-Bcl-2 antisense nucleotides" and "Bcl-2 inhibitors". The "anti-Bcl-2 antisense nucleotides" down-regulate the Bcl-2 mRNA levels and reduces Bcl-2 protein expression. Examples of such anti-Bcl-2 antisense nucleotides include Oblimersen and SPC-2996. The term "Bcl-2 inhibitors" as used herein refers to agents which inhibit the Bcl-2 protein interaction activity either by the inhibition of the phosphorylation of Bcl-2 ("Bcl-2 protein phosphorylation inhibitors") such as e.g. RTA-402 or by binding to the Bcl-2 protein and thus disruption of the Bad/Bcl-2 complex (these are referred to as "Bcl-2 protein binding inhibitors"). Preferably said Bcl-2 inhibitors are Bcl-2 protein binding inhibitors. The Bcl-2 inhibitory activity via direct binding of such Bcl-2 protein binding inhibitors can be measured via a competitive binding assay. Thus the IC50 of the inhibition of the Bcl-2 protein activity can be determined in an homogenous time resolved fluorescence (HTRF) Assay according to Example 3. Preferably the IC50 of anti-Bcl-2 inhibitory activity is 5$\mu$M or less, more preferably 1$\mu$M or less. Such Bcl-2 protein binding inhibitors include compounds such as Gossypol, AT-101, Obatoclax mesylate, A-371191, A-385358, A-438744, ABT-737, ABT-263, AT-101, BL-11, BL-193, GX-15-003, 2-Methoxyantimycin $A_3$, HA-14-1, KF-67544, Purpurogallin, TP-TW-37, YC-137 and Z-24, preferably ABT-263 and ABT-737.

Oblimersen is an antisense oligonucleotide that inhibits Bcl-2 expression. The antisense oligonucleotide, its sequence and its preparation are described e.g. in WO 95/08350, WO 1999/051259, WO 2002/017852, WO 2004/056971 and US 5,734,033. Oblimersen (or other synonyms: Genansense, G-3139, Oblimersen sodium) as used herein means Heptadecasodium salt of 18-mer antisense phosphorothioate oligodeoxynucleotide whose sequence is: 5'-TCTCCCAGCGTGCGCCAT-3'; Heptadecasodium salt of antisense oligonucleotide from fragment 32-49nt (start codon region) of the human BCL2 cDNA; d(P-thio)(T-C-T-C-C-C-A-G-C-G-T-G-C-G-C-C-A-T) DNA heptadecasodium salt; P-Thiothymidylyl-(3'--5')-2'-deoxy-P-thiocytidylyl-(3'-5')-P-thiothymidylyl-(3'--5')-2'-deoxy-P-thiocytidylyl-(3'-5')-2'-deoxy-P-thiocytidylyl-(3'-5')-2'-deoxy-P-thiocytidylyl-(3'-5')-2'-deoxy-P-thioadenylyl-(3'--5')-2'-deoxy-P-thioguanylyl-(3'-5')-2'-deoxy-P-thiocytidylyl-(3'-5')-2'-deoxy-P-thioguanylyl-(3'--5')-P-thiothymidylyl-(3'--5')-2'-deoxy-P-thioguanylyl-(3'--5')-2'-deoxy-P-thiocytidylyl-(3'-5')-2'-deoxy-P-thioguanylyl-(3'-5')-2'-deoxy-P-thiocytidylyl-(3'-5')-2'-deoxy-P-thiocytidylyl-(3'-5')-2'-deoxy-P-thioadenylyl-(3'--5')-thymidine heptadecasodium salt.

SPC-2996, an antisense oligonucleotide, is a 16-Mer antisense phosphorothioate oligonucleotides whose sequence is 5'-CTCCCAACGTGCGCCA-3' and in which nucleotides 1, 2, 14 and 15 are locked nucleic acid (LNA) nucleotides with enhanced resistance to nuclease digestion. This antisense LNA oligonucleotide targets nucleotides 33-48 (coding sequence) of human Bcl-2.

RTA-402 as used herein means CDDO-Me, the methyl ester of the C28-triterpenoid: oleanane triterpenoid 2-cyano-3,12-dioxoolean-1,9-dien-28-oic acid (CDDO) (See e.g. Honda, T., Rounds BV Bore, L., et al. J Med Chem. 43 (2000) 4233-4246), which blocks Bcl-2 protein phosphorylation (Konopleva, M., et al., Blood 99 (2002) 326-35).

ABT-737 as used herein means N-[4-[4-(4'-Chlorobiphenyl-2-ylmethyl)piperazin-1-yl]benzoyl]-3-[3-(dimethylamino)-1(R)-(phenylsulfanylmethyl)propylamino]-4-nitrobenzenesulfonamide; 4-[4-(4'-Chlorobipheriyl-2-ylmethyl)piperazin-1-yl]-N-[3-[3-(dimethylamino)-1(R)-(phenylsulfanylmethyl)propylamino]-4-nitrophenylsulfonyllbenzamide, a Bcl-2 inhibitor of formula I, which is described in WO 2006/099667 or Corey, S., et al., Cancer Cell 8 (2005) 5-6.

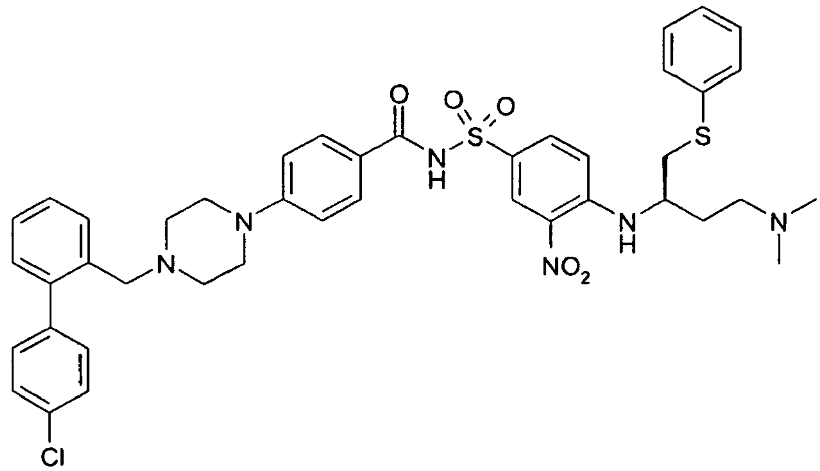

formula I.

ABT-263 as used herein means a Bcl-2 inhibitor of formula II, which is described in US 2007/027,135,

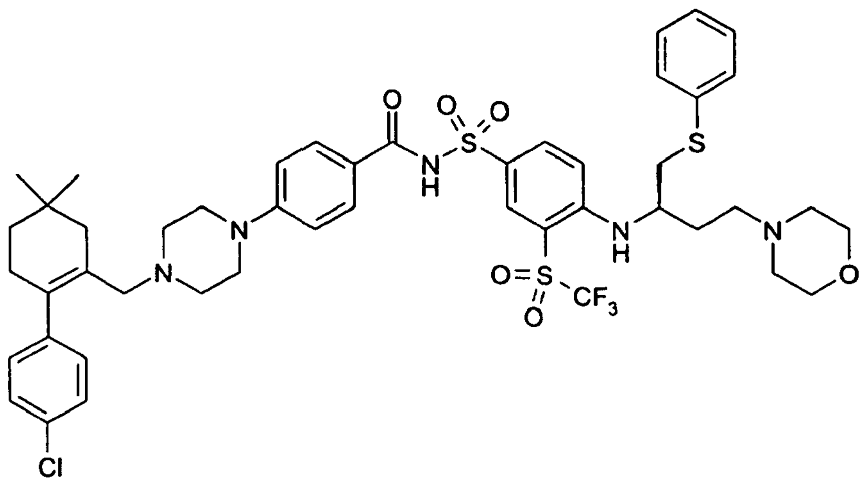

formula II.

A-371191 as used herein means a Bcl-2 inhibitor of formula III,

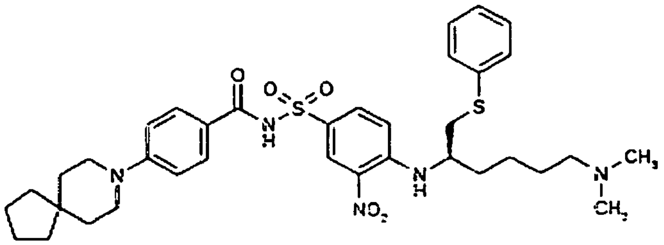

formula III.

A-385358 as used herein means [(R)-4-(3-dimethylamino-1-phenylsulfanylmethyl-propylamino)-N-[4-(4,4-dimethyl-piperidin-l-yl)-benzoyl]-3-nitrobenzenesulfonamide (as e.g. disclosed in Shoemaker, A.R., et al., Cancer Research 66 (2006) 8731-8739) a Bcl-2 inhibitor of formula IV,

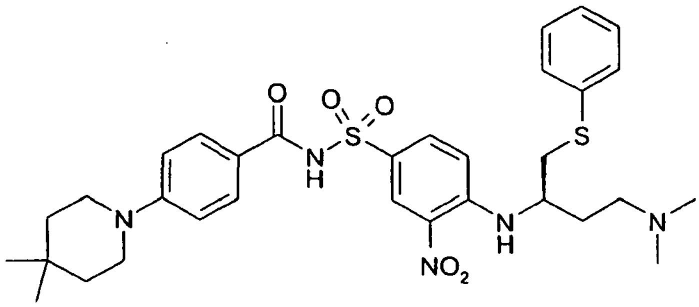

formula IV.

Gossypol as used herein means either a racemic mixture of (+)-Gossypol or (-)-Gossypol (a Bcl-2 inhibitor of formula V), or pure (+)-Gossypol or (-)-Gossypol, preferably Gossypol refers to pure (-)-Gossypol.

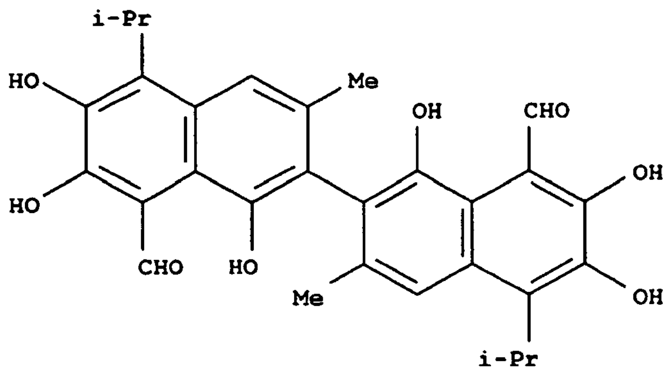

formula V.

AT-101 as used herein means clinical lead compound of Ascenta Therapeutics AT-101, a Bcl-2 inhibitor and derivative of R (-)-gossypol.

Obatoclax mesylate (or other synonyms: GX-015-070;or GX15-070) as used herein means 2-[2-(3,5-Dimethyl-1H-pyrrol-2-ylmethylene)-3-methoxy-2H-pyrrol-5-yl]-1H-indole methanesulfonate, a Bcl-2 inhibitor, which is described e.g. in WO 2004/106328, WO 2006/089397 and Walensky, L.D., Cell Death and Differentiation, 13 (2006)

1339-1350.

TW-37 as used herein means a Bcl-2 inhibitor of formula VI,

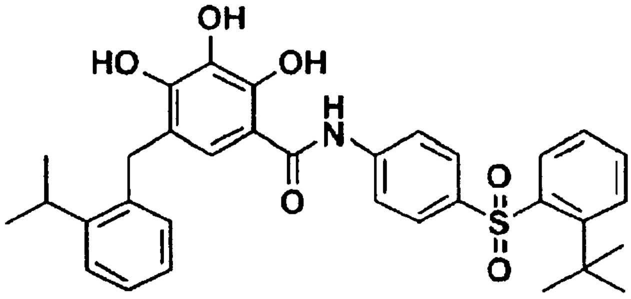

formula VI.

BL-193 as used herein means a Bcl-2 inhibitor of formula VII,

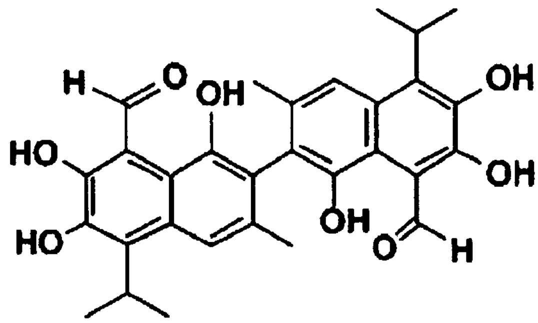

formula VII.

NSC-719664 as used herein means 2-Methoxy-Antimycin $A_3$, a Bcl-2 inhibitor derived from Antimycin $A_3$.

YC-137 is described e.g. in Walensky, L.D., Cell Death and Differentiation 13 (2006) 1339-1350.

Purpurogallin is described e.g. in Walensky, L.D., Cell Death and Differentiation 13 (2006) 1339-1350.

HA-14-1 is described e.g. in Walensky, L.D., Cell Death and Differentiation 13 (2006) 1339-1350.

Z-24 as used herein means 3*Z*-3-[(1*H*-pyrrol-2-yl)-methylidene]-1-(1-piperidinylmethyl)-1,3-2H-indol-2-one, a Bcl-2 inhibitor of formula VIII,

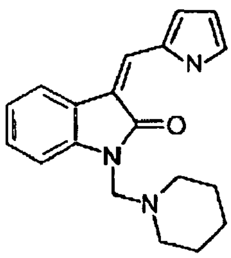

formula VIII.

Preferably the anti-Bcl-2 active agent is selected from Oblimersen, SPC-2996, RTA-402, Gossypol, AT-101, Obatoclax mesylate, A-371191, A-385358, A-438744, ABT-737, AT-101, BL-11, BL-193, GX-15-003, 2-Methoxyantimycin $A_3$, HA-14-1, KF-67544, Purpurogallin, TP-TW-37, YC-137 and Z-24.

Preferably the anti-Bcl-2 active agent is a Bcl-2 protein binding inhibitor with an IC50 of the anti-Bcl-2 inhibitory activity of 5 $\mu$M or less. Such Bcl-2 protein binding inhibitor is preferably selected from Gossypol, AT-101, Obatoclax mesylate, ABT-263 and ABT-737, more preferably from ABT-263 or ABT-737.

The term "expression of the CD20" antigen is intended to indicate an significant level of expression of the CD20 antigen in a cell, preferably on the cell surface of a T- or B- Cell, more preferably a B-cell, from a tumor or cancer, respectively, preferably a non-solid tumor. Patients having a "CD20 expressing cancer" can be determined by standard assays known in the art. E.g. CD20 antigen expression is measured using immunohistochemical (IHC) detection, FACS or via PCR-based detection of the corresponding mRNA.

The term "CD20 expressing cancer" as used herein refers to all cancers in which the cancer cells show an expression of the CD20 antigen. Such CD20 expressing cancer may be, for example, lymphomas, lymphocytic leukemias, lung cancer, non small cell lung (NSCL) cancer, bronchioloalviolar cell lung cancer, bone cancer, pancreatic cancer, skin cancer, cancer of the head or neck, cutaneous or intraocular melanoma, uterine cancer, ovarian cancer, rectal cancer, cancer of the anal region, stomach cancer, gastric cancer, colon cancer, breast cancer, uterine cancer, carcinoma of the fallopian tubes, carcinoma of the endometrium, carcinoma of the cervix, carcinoma of the vagina, carcinoma of the vulva, Hodgkin's Disease, cancer of the esophagus, cancer of the small intestine, cancer of the endocrine system, cancer of the thyroid gland, cancer of the parathyroid gland, cancer of the adrenal gland, sarcoma of soft tissue, cancer of the urethra, cancer of the penis, prostate cancer, cancer of the bladder, cancer of the kidney or ureter, renal cell carcinoma, carcinoma of the renal pelvis, mesothelioma, hepatocellular cancer, biliary cancer, neoplasms of the central nervous system (CNS), spinal axis tumors, brain stem glioma, glioblastoma multiforme, astrocytomas, schwanomas, ependymonas, medulloblastomas, meningiomas, squamous cell carcinomas, pituitary adenoma, including refractory versions of any of the above cancers, or a combination of one or more of the above cancers.

Preferably CD20 expressing cancer as used herein refers to lymphomas (preferably B-Cell Non-Hodgkin's lymphomas (NHL)) and lymphocytic leukemias. Such lymphomas and lymphocytic leukemias include e.g. a) follicular lymphomas, b) Small Non-Cleaved Cell Lymphomas/ Burkitt's lymphoma (including endemic Burkitt's lymphoma, sporadic Burkitt's lymphoma and Non-Burkitt's lymphoma) c) marginal zone lymphomas (including extranodal marginal zone B cell lymphoma (Mucosa-associated lymphatic tissue lymphomas, MALT), nodal marginal zone B cell lymphoma and splenic marginal zone lymphoma), d) Mantle cell lymphoma (MCL), e) Large Cell Lymphoma (including B-cell diffuse large cell lymphoma (DLCL), Diffuse Mixed Cell Lymphoma, Immunoblastic Lymphoma, Primary Mediastinal B-Cell Lymphoma, Angiocentric Lymphoma-Pulmonary B-Cell Lymphoma) f) hairy cell leukemia, g) lymphocytic lymphoma, waldenstrom's macroglobulinemia, h) acute lymphocytic leukemia (ALL), chronic lymphocytic leukemia (CLL)/ small lymphocytic lymphoma (SLL), B-cell prolymphocytic leukemia, i) plasma cell neoplasms, plasma cell myeloma, multiple myeloma, plasmacytoma j) Hodgkin's disease.

More preferably the CD20 expressing cancer is a B-Cell Non-Hodgkin's lymphomas (NHL). Especially the CD20 expressing cancer is a Mantle cell lymphoma (MCL), acute lymphocytic leukemia (ALL), chronic lymphocytic leukemia (CLL), B-cell diffuse large cell lymphoma (DLCL), Burkitt's lymphoma, hairy cell leukemia, follicular lymphoma, multiple myeloma, marginal zone lymphoma, post transplant lymphoproliferative disorder (PTLD), HIV associated lymphoma, waldenstrom's macroglobulinemia, or primary CNS lymphoma.

The term "treating" as used herein, unless otherwise indicated, means reversing, alleviating, inhibiting the progress of, or preventing, either partially or completely, the growth of tumors, tumor metastases, or other cancer-causing or neoplastic cells in a patient. The term "treatment" as used herein, unless otherwise indicated, refers to the act of treating.

The term "a method of treating" or its equivalent, when applied to, for example, cancer refers to a procedure or course of action that is designed to reduce or eliminate the number of cancer cells in a patient, or to alleviate the symptoms of a cancer. "A method of treating" cancer or another proliferative disorder does not necessarily mean that the cancer cells or other disorder will, in fact, be eliminated, that the number of cells or disorder will, in fact, be reduced, or that the symptoms of a cancer or other disorder will, in fact, be alleviated. Often, a method of treating cancer will be performed even with a low likelihood of success, but which, given the medical history and estimated survival expectancy of a patient, is nevertheless deemed to induce an overall beneficial course of action. The terms "co-administration" or "co-administering" refer to the administration of said type II anti-CD20 antibody and said Bcl-2 inhibitor as one single formulation or as two separate formulations. The co-administration can be simultaneous or sequential in either order, wherein preferably there is a time period while both (or all) active agents simultaneously exert their biological activities. Said type II anti-CD20 antibody and said Bcl-2 inhibitor are co-administered either simultaneously or sequentially (e.g. via an intravenous (i.v.) through a continuous infusion (one for the antibody and eventually one for the Bcl-2 inhibitor; or the Bcl-2 inhibitor is administered orally). When both therapeutic agents are co-administered sequentially the dose is administered either on the same day in two separate administrations, or one of the agents is administered on day 1 and the second is co-administered on day 2 to day 7, preferably on day 2 to 4. Thus the term "sequentially" means within 7 days after the dose of the first antibody, preferably within 4 days after the dose of the first antibody; and the term "simultaneously" means at the same time. The terms "co-administration" with respect to the maintenance doses of the type II anti-CD20 antibody and the Bcl-2 inhibitor mean that the maintenance doses can be either co-administered

simultaneously, if the treatment cycle is appropriate for both drugs, e.g. every week. Or the Bcl-2 inhibitor is e.g. administered e.g. every first to third day and type II anti-CD20 antibody is administered every week. Or the maintenance doses are co-administered sequentially, either within one or within several days.

It is self-evident that the antibodies are administered to the patient in a "therapeutically effective amount" (or simply "effective amount") which is the amount of the respective compound or combination that will elicit the biological or medical response of a tissue, system, animal or human that is being sought by the researcher, veterinarian, medical doctor or other clinician.

The amount of co-administration of said type II anti-CD20 antibody and said Bcl-2 inhibitor and the timing of co-administration will depend on the type (species, gender, age, weight, etc.) and condition of the patient being treated and the severity of the disease or condition being treated. Said type II anti-CD20 antibody and said Bcl-2 inhibitor are suitably co-administered to the patient at one time or over a series of treatments. Depending on the type and severity of the disease, about 1 μg /kg to 50 mg/kg (e.g. 0.1-20 mg/kg) of said type II anti-CD20 antibody and 1 mg /kg to 200 mg/kg (e.g. 10-150 mg/kg) of said Bcl-2 inhibitor is an initial candidate dosage for co-administration of both drugs to the patient. If the administration is intravenous the initial infusion time for said type II anti-CD20 antibody or said Bcl-2 inhibitor may be longer than subsequent infusion times, for instance approximately 90 minutes for the initial infusion, and approximately 30 minutes for subsequent infusions (if the initial infusion is well tolerated).

The preferred dosage of said type II anti-CD20 antibody will be in the range from about 0.05mg/kg to about 30mg/kg. Thus, one or more doses of about 0.5mg/kg, 2.0mg/kg, 4.0mg/kg, 10 mg/kg or 30mg/kg (or any combination thereof) may be co-administered to the patient. The preferred dosage of said Bcl-2 inhibitor will be in the range from 20 mg/kg to about 150mg/kg. Depending on the on the type (species, gender, age, weight, etc.) and condition of the patient and on the type of anti-CD20 antibody and Bcl-2 inhibitor, the dosage and the administration schedule of said anti-CD20 antibody can differ from the dosage of Bcl-2 inhibitor. E.g. the said anti-CD20 antibody may be administered e.g. every one to three weeks and said Bcl-2 inhibitor may be administered daily or every 2 to 7 days. An initial higher loading dose, followed by one or more lower doses may also be administered.

In a preferred embodiment, the medicament is useful for preventing or reducing metastasis or further dissemination in such a patient suffering from CD20 expressing cancer. The medicament is useful for increasing the duration of survival of such a patient, increasing the progression free survival of such a patient, increasing the duration of response, resulting in a statistically significant and clinically meaningful improvement of the treated patient as measured by the duration of survival, progression free survival, response rate or duration of response. In a preferred embodiment, the medicament is useful for increasing the response rate in a group of patients.

In the context of this invention, additional other cytotoxic, chemotherapeutic or anti-cancer agents, or compounds that enhance the effects of such agents (e.g. cytokines) may be used in the type II anti-CD20 antibody and Bcl-2 inhibitor combination treatment of CD20 expressing cancer. Such molecules are suitably present in combination in amounts that are effective for the purpose intended. Preferably the type II anti-CD20 antibody and Bcl-2 inhibitor combination treatment is used without such additional cytotoxic, chemotherapeutic or anti-cancer agents, or compounds that enhance the effects of such agents.

Such agents include, for example: alkylating agents or agents with an alkylating action, such as cyclophosphamide (CTX; e.g. cytoxan®), chlorambucil (CHL; e.g. leukeran®), cisplatin (CisP; e.g. platinol®) busulfan (e.g. myleran®), melphalan, carmustine (BCNU), streptozotocin, triethylenemelamine (TEM), mitomycin C, and the like; anti-metabolites, such as methotrexate (MTX), etoposide (VP16; e.g. vepesid®), 6-mercaptopurine (6MP), 6-thiocguanine (6TG), cytarabine (Ara-C), 5-fluorouracil (5-FU), capecitabine (e.g. Xeloda®), dacarbazine (DTIC), and the like; antibiotics, such as actinomycin D, doxorubicin (DXR; e.g. adriamycin®), daunorubicin (daunomycin), bleomycin, mithramycin and the like; alkaloids, such as vinca alkaloids such as vincristine (VCR), vinblastine, and the like; and other antitumor agents, such as paclitaxel (e.g. taxol®) and paclitaxel derivatives, the cytostatic agents, glucocorticoids such as dexamethasone (DEX; e.g. decadron®) and corticosteroids such as prednisone, nucleoside enzyme inhibitors such as hydroxyurea, amino acid depleting enzymes such as asparaginase, leucovorin and other folic acid derivatives, and similar, diverse antitumor agents. The following agents may also be used as additional agents: arnifostine (e.g. ethyol®), dactinomycin, mechlorethamine (nitrogen mustard), streptozocin, cyclophosphamide, lomustine (CCNU), doxorubicin lipo (e.g. doxil®), gemcitabine (e.g. gemzar®), daunorubicin lipo (e.g. daunoxome®), procarbazine, mitomycin, docetaxel (e.g. taxotere®), aldesleukin, carboplatin, oxaliplatin, cladribine, camptothecin, CPT 11 (irinotecan), 10-hydroxy 7-ethyl-camptothecin (SN38), floxuridine, fludarabine, ifosfamide, idarubicin, mesna, interferon beta, interferon alpha, mitoxantrone, topotecan, leuprolide, megestrol, melphalan, mercaptopurine, plicamycin, mitotane, pegaspargase, pentostatin, pipobroman, plicamycin, tamoxifen, teniposide, testolactone, thioguanine, thiotepa, uracil mustard, vinorelbine, chlorambucil. Preferably the type II anti-CD20 antibody and Bcl-2 inhibitor combination treatment is used without such additional agents.

The use of the cytotoxic and anticancer agents described above as well as antiproliferative target-specific anticancer drugs like protein kinase inhibitors in chemotherapeutic regimens is generally well characterized in the cancer therapy arts, and their use herein falls under the same considerations for monitoring tolerance and effectiveness and for controlling administration routes and dosages, with some adjustments. For example, the actual dosages of the

cytotoxic agents may vary depending upon the patient's cultured cell response determined by using histoculture methods. Generally, the dosage will be reduced compared to the amount used in the absence of additional other agents.

Typical dosages of an effective cytotoxic agent can be in the ranges recommended by the manufacturer, and where indicated by in vitro responses or responses in animal models, can be reduced by up to about one order of magnitude concentration or amount. Thus, the actual dosage will depend upon the judgment of the physician, the condition of the patient, and the effectiveness of the therapeutic method based on the in vitro responsiveness of the primary cultured malignant cells or histocultured tissue sample, or the responses observed in the appropriate animal models.

In the context of this invention, an effective amount of ionizing radiation may be carried out and/or a radiopharmaceutical may be used in addition to the type II anti-CD20 antibody and Bcl-2 inhibitor combination treatment of CD20 expressing cancer. The source of radiation can be either external or internal to the patient being treated. When the source is external to the patient, the therapy is known as external beam radiation therapy (EBRT). When the source of radiation is internal to the patient, the treatment is called brachytherapy (BT). Radioactive atoms for use in the context of this invention can be selected from the group including, but not limited to, radium, cesium-137, iridium-192, americium-241, gold-198, cobalt-57, copper-67, technetium-99, iodine-123, iodine-131, and indium-111. Is also possible to label the antibody with such radioactive isotopes. Preferably the type II anti-CD20 antibody and Bcl-2 inhibitor combination treatment is used without such ionizing radiation.

Radiation therapy is a standard treatment for controlling unresectable or inoperable tumors and/or tumor metastases. Improved results have been seen when radiation therapy has been combined with chemotherapy. Radiation therapy is based on the principle that high-dose radiation delivered to a target area will result in the death of reproductive cells in both tumor and normal tissues. The radiation dosage regimen is generally defined in terms of radiation absorbed dose (Gy), time and fractionation, and must be carefully defined by the oncologist. The amount of radiation a patient receives will depend on various considerations, but the two most important are the location of the tumor in relation to other critical structures or organs of the body, and the extent to which the tumor has spread. A typical course of treatment for a patient undergoing radiation therapy will be a treatment schedule over a 1 to 6 week period, with a total dose of between 10 and 80 Gy administered to the patient in a single daily fraction of about 1.8 to 2.0 Gy, 5 days a week. In a preferred embodiment of this invention there is synergy when tumors in human patients are treated with the combination treatment of the invention and radiation. In other words, the inhibition of tumor growth by means of the agents comprising the combination of the invention is enhanced when combined with radiation, optionally with additional chemotherapeutic or anticancer agents. Parameters of adjuvant radiation therapies are, for example, contained in WO 99/60023.

The type II anti-CD20 antibodies are administered to a patient according to known methods, by intravenous administration as a bolus or by continuous infusion over a period of time, by intramuscular, intraperitoneal, intracerobrospinal, subcutaneous, intra-articular, intrasynovial, or intrathecal routes. Intravenous or subcutaneous administration of the antibodies is preferred.

The Bcl-2 inhibitors are administered to a patient according to known methods, e.g. by intravenous administration as a bolus or by continuous infusion over a period of time, by intramuscular, intraperitoneal, intracerobrospinal, subcutaneous, intra-articular, intrasynovial, intrathecal, or peroral routes. Intravenous, subcutaneous or oral administration of the Bcl-2 inhibitors is preferred.

The invention further comprises a kit comprising a type II anti-CD20 antibody and an anti-Bcl-2 active agent for the combination treatment of a patient suffering from a CD20 expressing cancer. In a preferred embodiment, the kit containers may further include a pharmaceutically acceptable carrier. The kit may further include a sterile diluent, which is preferably stored in a separate additional container. The kit may further include a package insert comprising printed instructions directing the use of the combined treatment as a method for a CD20 expressing cancer disease, preferably a B-Cell Non-Hodgkin's lymphoma (NHL).

The term "package insert" refers to instructions customarily included in commercial packages of therapeutic products, which may include information about the indications, usage, dosage, administration, contraindications and/or warnings concerning the use of such therapeutic products.

In a preferred embodiment, the article of manufacture containers may further include a pharmaceutically acceptable carrier. The article of manufacture may further include a sterile diluent, which is preferably stored in a separate additional container.

As used herein, a "pharmaceutically acceptable carrier" is intended to include any and all material compatible with pharmaceutical administration including solvents, dispersion media, coatings, antibacterial and antifungal agents, isotonic and absorption delaying agents, and other materials and compounds compatible with pharmaceutical administration. Except insofar as any conventional media or agent is incompatible with the active compound, use thereof in the compositions of the invention is contemplated. Supplementary active compounds can also be incorporated into the compositions.

Pharmaceutical Compositions:

Pharmaceutical compositions can be obtained by processing the type II anti-CD20 antibody and/or the anti-Bcl-2 active agent according to this invention with pharmaceutically acceptable, inorganic or organic carriers. Lactose, corn starch or derivatives thereof, talc, stearic acids or it's salts and the like can be used, for example, as such carriers for tablets, coated tablets, dragées and hard gelatine capsules. Suitable carriers for soft gelatine capsules are, for example, vegetable oils, waxes, fats, semi-solid and liquid polyols and the like. Depending on the nature of the active substance no carriers are, however, usually required in the case of soft gelatine capsules. Suitable carriers for the production of solutions and syrups are, for example, water, polyols, glycerol, vegetable oil and the like. Suitable carriers for suppositories are, for example, natural or hardened oils, waxes, fats, semi-liquid or liquid polyols and the like.

The pharmaceutical compositions can, moreover, contain preservatives, solubilizers, stabilizers, wetting agents, emulsifiers, sweeteners, colorants, flavorants, salts for varying the osmotic pressure, buffers, masking agents or antioxidants. They can also contain still other therapeutically valuable substances.

One embodiment of the invention is pharmaceutical composition comprising both said type II anti-CD20 antibody and said anti-Bcl-2 active agent, in particular for use in CD20 expressing cancer.

Said pharmaceutical composition may further comprise one or more pharmaceutically acceptable carriers.

The present invention further provides a pharmaceutical composition, in particular for use in cancer, comprising (i) an effective first amount of a type II anti-CD20 antibody , and (ii) an effective second amount of an anti-Bcl-2 active agent. Such composition optionally comprises pharmaceutically acceptable carriers and / or excipients.

Pharmaceutical compositions of the type II anti-CD20 antibody alone used in accordance with the present invention are prepared for storage by mixing an antibody having the desired degree of purity with optional pharmaceutically acceptable carriers, excipients or stabilizers (Remington's Pharmaceutical Sciences 16th edition, Osol, A. Ed. (1980)), in the form of lyophilized formulations or aqueous solutions. Acceptable carriers, excipients, or stabilizers are nontoxic to recipients at the dosages and concentrations employed, and include buffers such as phosphate, citrate, and other organic acids; antioxidants including ascorbic acid and methionine; preservatives (such as octadecyldimethylbenzyl ammonium chloride; hexamethonium chloride; benzalkonium chloride, benzethonium chloride; phenol, butyl or benzyl alcohol; alkyl parabens such as methyl or propyl paraben; catechol; resorcinol; cyclohexanol; 3-pentanol; and m-cresol); low molecular weight (less than about 10 residues) polypeptides; proteins, such as serum albumin, gelatin, or immunoglobulins; hydrophilic polymers such as polyvinylpyrrolidone; amino acids such as glycine, glutamine, asparagine, histidine, arginine, or lysine; monosaccharides, disaccharides, and other carbohydrates including glucose, mannose, or dextrins; chelating agents such as EDTA; sugars such as sucrose, mannitol, trehalose or sorbitol; salt-forming counter-ions such as sodium; metal complexes (e.g. Zn-protein complexes); and/or non-ionic surfactants such as TWEEN™, PLURONICS™ or polyethylene glycol (PEG).

Pharmaceutical compositions of the anti-Bcl-2 active agent alone, e.g. the Bcl-2 inhibitor, depend on their pharmaceutical properties; e.g. for small chemical compounds such as e.g. ABT-737 or ABT-263, one formulation could be e.g. the following:

a) Tablet Formulation (Wet Granulation):

| Item | Ingredients | mg/tablet | | | |
|---|---|---|---|---|---|
| 1. | Compound of formula (I) | 5 | 25 | 100 | 500 |
| 2. | Lactose Anhydrous DTG | 125 | 105 | 30 | 150 |
| 3. | Sta-Rx 1500 | 6 | 6 | 6 | 30 |
| 4. | Microcrystalline Cellulose | 30 | 30 | 30 | 150 |
| 5. | Magnesium Stearate | 1 | 1 | 1 | 1 |
| | Total | 167 | 167 | 167 | 831 |

Manufacturing Procedure:

1. Mix items 1, 2, 3 and 4 and granulate with purified water.

2. Dry the granules at 50°C.
3. Pass the granules through suitable milling equipment.
4. Add item 5 and mix for three minutes; compress on a suitable press.

b) Capsule Formulation:

| Item | Ingredients | mg/capsule | | | |
|---|---|---|---|---|---|
| 1. | Compound of formula (I) | 5 | 25 | 100 | 500 |
| 2. | Hydrous Lactose | 159 | 123 | 148 | --- |
| 3. | Corn Starch | 25 | 35 | 40 | 70 |
| 4. | Talc | 10 | 15 | 10 | 25 |
| 5. | Magnesium Stearate | 1 | 2 | 2 | 5 |
| | Total | 200 | 200 | 300 | 600 |

Manufacturing Procedure:

1. Mix items 1, 2 and 3 in a suitable mixer for 30 minutes.
2. Add items 4 and 5 and mix for 3 minutes.
3. Fill into a suitable capsule.

In one further embodiment of the invention the pharmaceutical compositions according to the invention are two separate formulations for said type II anti-CD20 antibody and said Bcl-2 inhibitor.

The active ingredients may also be entrapped in microcapsules prepared, for example, by coacervation techniques or by interracial polymerization, for example, hydroxymethylcellulose or gelatin-microcapsules and poly- (methylmethacylate) microcapsules, respectively, in colloidal drug delivery systems (for example, liposomes, albumin microspheres, microemulsions, nano- particles and nanocapsules) or in macroemulsions. Such techniques are disclosed in Remington's Pharmaceutical Sciences 16th edition, Osol, A. Ed. (1980).

Sustained-release preparations may be prepared. Suitable examples of sustained-release preparations include semipermeable matrices of solid hydrophobic polymers containing the antibody, which matrices are in the form of shaped articles, e.g. films, or microcapsules. Examples of sustained-release matrices include polyesters, hydrogels (for example, poly(2-hydroxyethyl-methacrylate), or poly(vinylalcohol)), polylactides (US 3,773,919), copolymers of L-glutamic acid and gamma-ethyl-L-glutamate, non-degradable ethylene-vinyl acetate, degradable lactic acid-glycolic acid copolymers such as the LUPRON DEPOT™ (injectable microspheres composed of lactic acid-glycolic acid copolymer and leuprolide acetate), and poly-D-(-)-3-hydroxybutyric acid.

The formulations to be used for in vivo administration must be sterile. This is readily accomplished by filtration through sterile filtration membranes.

The present invention further provides a method for the treatment of cancer, comprising administering to a subject in need of such treatment (i) an effective first amount of a type II anti-CD20 antibody ; and (ii) an effective second amount of an anti-Bcl-2 active agent.

The present invention further provides a method for the treatment of cancer, comprising administering to a subject in need of such treatment (i) an effective first amount of a type II anti-CD20 antibody ; and (ii) an effective second amount of an anti-Bcl-2 active agent.

As used herein, the term "patient" preferably refers to a human in need of treatment with type II anti-CD20 antibody (e.g. a patient suffering from CD20 expressing cancer) for any purpose, and more preferably a human in need of such a treatment to treat cancer, or a precancerous condition or lesion. However, the term "patient" can also refer to non-human animals, preferably mammals such as dogs, cats, horses, cows, pigs, sheep and non-human primates, among others.

The invention further comprises a type II anti-CD20 antibody for the treatment of CD20 expressing cancer in combination with an anti-Bcl-2 active agent.

The invention further comprises a type II anti-CD20 antibody for the treatment of a patient suffering from a

CD20 expressing cancer in combination an anti-Bcl-2 active agent.

The invention further comprises a type II anti-CD20 antibody and an anti-Bcl-2 active agent for use in the treatment of CD20 expressing cancer.

The invention further comprises a type II anti-CD20 antibody and an anti-Bcl-2 active agent for use in the treatment of a patient suffering from a CD20 expressing cancer.

Preferably said anti-Bcl-2 active agent is selected from Oblimersen, SPC-2996, RTA-402, Gossypol, AT-101, Obatoclax mesylate, A-371191, A-385358, A-438744, ABT-737, AT-101, BL-11, BL-193, GX-15-003, 2-Methoxyantimycin $A_3$, HA-14-1, KF-67544, Purpurogallin, TP-TW-37, YC-137 and Z-24.

Preferably the anti-Bcl-2 active agent is a Bcl-2 protein binding inhibitor with an IC50 of the anti-Bcl-2 inhibitory activity of 5 μM or less. Such Bcl-2 protein binding inhibitor is preferably selected from Gossypol, AT-101, Obatoclax mesylate, ABT-263 and ABT-737, more preferably from ABT-263 or ABT-737.

Preferably said type II anti-CD20 antibody has a ratio of the binding capacities to CD20 on Raji cells (ATCC-No. CCL-86) of said type II anti-CD20 antibody compared to rituximab of 0.3 to 0.6, more preferably 0.35 to 0.55, and still more preferably 0.4 to 0.5.

Preferably said type II anti-CD20 antibody is a humanized B-Ly1 antibody.

Preferably said type II anti-CD20 antibody has increased antibody dependent cellular cytotoxicity (ADCC).

Preferably the CD20 expressing cancer is a B-Cell Non-Hodgkin's lymphoma (NHL).

Preferably said type II anti-CD20 antibody is a monoclonal antibody.

The following examples, sequence listing and figures are provided to aid the understanding of the present invention, the true scope of which is set forth in the appended claims. It is understood that modifications can be made in the procedures set forth without departing from the spirit of the invention.

<u>Sequence Listing</u>

| | |
|---|---|
| SEQ ID NO: 1 | amino acid sequence of variable region of the heavy chain (VH) of murine monoclonal anti-CD20 antibody B-Ly1. |
| SEQ ID NO: 2 | amino acid sequence of variable region of the light chain (VL) of murine monoclonal anti-CD20 antibody B-Ly1. |
| SEQ ID NO: 3 -19 | amino acid sequences of variable region of the heavy chain (VH) of humanized B-Ly1 antibodies (B-HH2 to B-HH9, B-HL8, and B-HL10 to B-HL17) |
| SEQ ID NO: 20 | amino acid sequences of variable region of the light chain (VL) of humanized B-Ly1 antibody B-KV1 |

<u>Description of the Figures</u>

| | |
|---|---|
| Figure 1 | Antitumor activity of combined treatment of a type II anti-CD20 antibody (B-HH6-B-KV1 GE) having a ratio of the binding capacities to CD20 on Raji cells (ATCC-No. CCL-86) of said type II anti-CD20 antibody compared to rituximab of 0.44, with a Bcl-2 inhibitor (ABT-737) (Bcl-2 Inhibitory Activity of IC50: 0.040 μM) on SU-DHL-4 DLBCL B-Cell Non-Hodgkin-Lymphoma (NHL). Mean values of tumor volume [$mm^3$] plotted on the y-axis; number of days after injection of tumor cells plotted on the x-axis. Legend: A)Vehicle (*circles* ), B) humanized B-ly1 (B-HH6-B-KV1 GE) 10 mg/kg once weekly *(squares),* C) Bcl-2 inhibitor ABT-737 100 mg/kg every second day *(triangles)* and D) humanized B-ly1 (B-HH6-B-KV1 GE) 10 mg/kg once weekly co-administered with Bcl-2 inhibitor ABT-737 (100 mg/kg every second day) (crosses) |
| Figure 2 | Mean Fluorescence Intensity (MFI, left y-axis) of type I anti-CD20 antibody (Cy5-rituximab = white bar) and type II anti-CD20 antibody (Cy5 humanized B-Ly1 B-HH6-B-KV1 GE = black bar) on Raji cells (ATCC-No. CCL-86) ; Ratio of the binding capacities to CD20 of type I anti-CD20 antibody (rituximab) and type II anti-CD20 antibody (B-HH6-B-KV1 GE) compared to rituximab (scaled on right y-axis) |
| Figure 3 | Antitumor activity of treatment of two type II anti-CD20 antibodies on the Z138 human Non-Hodgkin-Lymphoma (NHL). Both antibodies are humanized B-Ly1 anti-CD20 antibodies; 1) B-HH6-B-KV1glycoengineered (GE) and 2) B-HH6-B-KV1 wildtype (wt, non-glycoengineered). Mean values of tumor volume [$mm^3$] plotted on the y-axis; number of days after injection of tumor cells plotted on the x-axis. Legend: A)Vehicle (*circles*), B) humanized B-Ly1 GE (B-HH6-B-KV1 GE) 30 mg/kg once weekly *(triangles)* and C) humanized B-Ly1 wt (B-HH6-B-KV1 wt) 30 mg/kg once weekly (crosses) |

Experimental Procedures

Example 1

Antitumor activity of combined treatment of a type II anti-CD20 antibody (B-HH6-B-KV1 GE) with a Bcl-2 inhibitor (ABT-737)

Test agents

Type II anti-CD20 antibody B-HH6-B-KV1 GE (= humanized B-Ly1, glycoengineered B-HH6-B-KV1, see WO 2005/044859 and WO 2007/031875) was provided as stock solution (c=9.4 mg/ml) from GlycArt, Schlieren, Switzerland. Antibody buffer included histidine, trehalose and polysorbate 20. Antibody solution was diluted appropriately in PBS from stock for prior injections.

Bcl-2 inhibitor ABT-737 was provided as chemical powder and formulated in 1.5% DMSO, 5% Tween 80, 30% 1,2-Propanediol in 5% Glucose solution with c=10 mg/ml.

Cell lines and culture conditions

SU-DHL-4 human Non-Hodgkin-Lymphoma (NHL) cells (Chang, H., et al., Leuk. Lymphoma.8 (1992) 129-136) were kindly provided from DSMZ, Braunschweig. Tumor cell line was routinely cultured in RPMI medium (PAA, Laboratories, Austria) supplemented with 10 % fetal bovine serum (PAA Laboratories, Austria) and 2 mM L-glutamine, at 37 °C in a water-saturated atmosphere at 5 % $CO_2$. Passage 5 was used for transplantation.

Animals

Female SCID beige mice; age 4-5 weeks at arrival (purchased from Bomholtgard, Ry, Denmark) were maintained under specific-pathogen-free condition with daily cycles of 12 h light /12 h darkness according to committed guidelines (GV-Solas; Felasa; TierschG). Experimental study protocol was reviewed and approved by local government. After arrival animals were maintained in the quarantine part of the animal facility for one week to get accustomed to new environment and for observation. Continuous health monitoring was carried out on regular basis. Diet food (Provimi Kliba 3337) and water (acidified pH 2.5-3) were provided ad libitum.

Monitoring

Animals were controlled daily for clinical symptoms and detection of adverse effects. For monitoring throughout the experiment body weight of animals was documented two times weekly and tumor volume was measured by caliper after staging.

Treatment of animals

Animal treatment started at day of randomisation, 22 days after cell transplantation. Humanized type II anti-CD20 antibody B-HH6-B-KV1GE receiving groups as single agent or in combination and the corresponding vehicle group were treated i.v. q7d on study day 22, 29, 36 and 43 at the indicated dosage of 10 mg/kg. Bcl-2 inhibitor ABT-737 was given i.p. every second day (day 23-33, q2d,) at 100 mg/kg and due to low tolerability until day 41 at reduced dose of 50 mg/kg.

Tumor growth inhibition study in vivo

Tumor bearing animals receiving vehicle control had to be excluded 15 days after treatment initiation due to tumor burden. Treatment of animals with weekly B-HH6-B-KV1 GE (10 mg/kg) once weekly as single agent significantly inhibited xenograft growth for 14 days (TGI 87%) compared to control. However, despite weekly antibody treatments SU-DHL-4 xenografts continuously progressed. In contrast single agent therapy with bcl-2 inhibitor given every second day at 100 mg/kg was only slightly active and tumors grow progressively similar to control. Despite the moderate activity of both compounds as single agents, SU-DHL-4 lymphoma xenografts were forced to undergo complete remission in combination. Weekly treatment with B-HH6-B-KV1GE (10 mg/kg) and injection of Bcl-2 inhibitor ABT-737 every second day caused lymphoma regression within first week and in subsequent combination treatment period all SU-DHL-4 tumors showed complete tumor remission with no regrow observed.

Example 2

Determination of the ratio of the binding capacities to CD20 on Raji cells (ATCC-No. CCL-86) of type II anti-CD20 antibody compared to rituximab

Raji cells (ATCC-No. CCL-86) were maintained in culture in RPMI-1640 medium (PanBiotech GmbH, Cat.-No. PO4-18500) containing 10% FCS (Gibco, Cat.-No.10500-064). The type II anti-CD20 antibody B-HH6-B-KV1(humanized B-Ly1 antibody) and rituximab were labeled using Cy5 Mono NHS ester (Amersham GE Healthcare, Catalogue No. PA15101) according to the manufacturer's instructions. Cy5-conjugated rituximab had a labeling ratio of 2.0 molecules Cy5 per antibody. Cy5-conjugated B-HH6-B-KV1 had a labeling ratio of 2.2 molecules Cy5 per antibody. In order to determine and compare the binding capacities and mode of both antibodies, binding curves (by titration of Cy5-conjugated Rituximab and Cy5-conjugated B-HH6-B-KV1) were generated by direct immunofluorescence using the Burkitt's lymphoma cell line Raji (ATCC-No. CCL-86). Mean fluorescence intensities (MFI) for were analyzed as EC50 (50% of maximal intensity) for Cy5-conjugated Rituximab and Cy5-conjugated B-HH6-B-KV1, respectively. 5*105 cells per sample were stained for 30 min at 4 °C. Afterwards, cells were washed in culture medium. Propidium iodide (PI) staining was used to exclude dead cells. Measurements were performed using the FACSArray (Becton Dickinson), Propidium iodide (PI) was measured at Far Red A and Cy5 at Red-A. Figure 2 shows Mean Fluorescence Intensity (MFI) for binding at EC50 (50% of maximal intensity) of Cy5-labeled B-HH6-B-KV1(black bar) and Cy5-labeled rituximab (white bar).

Then the ratio of the binding capacities to CD20 on Raji cells (ATCC-No. CCL-86) is calculated according to the following formula:

$$\text{Ratio of the binding capacities to CD20 on Raji cells (ATCC-No. CCL-86)} =$$

$$\frac{\text{MFI(Cy5-anti-CD20 antibody)}}{\text{MFI(Cy5-rituximab)}} \times \frac{\text{Cy5labeling ratio(Cy5-rituximab)}}{\text{Cy5labeling ratio(Cy5-anti-CD20 antibody)}}$$

$$= \frac{\text{MFI(B-HH6-B-KV1)}}{\text{MFI(Cy5-rituximab)}} \times \frac{\text{Cy5labeling ratio(Cy5-rituximab)}}{\text{Cy5labeling ratio(B-HH6-B-KV1)}}$$

$$= \frac{207}{433} \times \frac{2.2}{2.0} = 0.44$$

Thus B-HH6-B-KV1 as a typical type II anti-CD20 antibody shows reduces binding capacity compared to rituximab.

Example 3

Determination of the IC50 value of the anti-Bcl-2 inhibitory activity of a Bcl-2 inhibitor (ABT-737)

Bcl-2 and Bcl-xL binding- HTRF assay procedures:

Compound preparation plate:

Compounds are serially diluted (3 fold, 10 point) starting at 1.8mM from a 10mM stock in 100% DMSO.

Reagents:

Bcl-2 Assay

1) Biotinylated-BAD peptide (Bio-BAD) (BAD = Bcl-2-antagonist of cell death; the BAD protein is an apoptosis inducer associated with BCL2 and BAX )) for Bcl-2 assay:

- prepare Bio-BAD peptide (73.64nM) in assay buffer containing 50mM Tris-HCL buffer, bovine serum albumin (BSA) 0.2mg/mL, Dithiothreitol 1 mM and 9% DMSO.

2) His6-Bcl2 :

- prepare His6-Bcl2 (180nM) in assay buffer containing 50mM Tris-HCL, bovine serum albumin (BSA) 0.2mg/mL, Dithiothreitol 1mM.

3) Lance Europium -Streptavidin (EU-SA) and Anti-6His APC

- prepare solution in detection buffer 50mM Tris-HCL, BSA 0.2mg/mL, Eu-SA 4.5nM and Anti-6His APC 67.5nM.

Final assay concentrations: Bio-BAD (22.5nM), His6-Bc-12 (80nM), EU-SA (1nM), APC (15nM)

Bcl-xL

1) Biotinylated-BAD peptide (Bio-BAD) for Bcl-xL assay:

- prepare Bio-BAD peptide (9.82nM) in assay buffer containing 50mM Tris-HCL, BSA 0.2mg/mL, Dithiothreitol 1mM and 9% DMSO.

2) HisBcl-xL :

- prepare His6-Bcl-xL (22.5nM) in assay buffer containing 50mM Tris-HCL buffer, BSA 0.2mg/mL, Dithiothreitol 1mM.

3) Lance Europium -Streptavidin (EU-SA) and Anti-6His APC

- prepare solution in detection buffer 50mM Tris-HCL, BSA 0.2mg/mL, Eu-SA 3.4nM and Anti-6His APC 45nM.

Final assay concentrations: Bio-Bad (3nM), His6-Bcl-xL (10nM), EU-SA (0.75nM), Anti-6His APC (10nM)

Procedure:

Transfer plate: transfer 5 μL of compound from compound prep plate (or 5 μL of 100% DMSO into no drug control wells) into a 384-well plate transfer plate and add 55μLs of Bio-BAD solution. Transfer 12 μL from the transfer plate into the assay plate and add 16 μL of either His6-Bcl2 or His6-BclXL for test wells or assay buffer for blanks. Incubate for 1 hour at 37C° . Add 8μLs of EU-SA/APC solution/well and incubate for 1 hour at room temperature. Plates are read on a plate reader suitable for homogenous time resolved fluorescence (HTRF) format at 340 nm excitation and 665/615 nm emission.
Final compound concentrations: 50, 16.7, 5.6, 1.85, 0.62, 0.21, 0.07, 0.03, 0.01, 0.004 μM.
Cross talk correction: Add into multiple wells 16 μL of assay buffer, 12 μL Bio-BAD, 8 μL of detection buffer with and without EU-SA/APC.
Result: ABT-737 was tested for Bcl-2 and Bcl-xL inhibition ; the $IC_{50}$ values were calculated using a non-linear curve fit (XLfit software (ID Business Solution Ltd., Guilford, Surrey, UK))
IC50 (Bcl-2) of ABT-737: 0.040 μM
IC50 (Bcl-xL) of ABT-737: 0.019 μM

<u>Example 4</u>

Similar antitumor activity of glycoengineered (GE) and non-glycoengineered (wildtype, wt) anti-CD20 antibody (B-HH6-B-KV1 GE and wt) against Z138 MCL xenografts in SCID beige mice

Test agents

Type II anti-CD20 antibody B-HH6-B-KV1 (glycoengineered (GE) and wildtype (wt)) were provided as stock

solution (c=9.4 mg/ml and 12.5 mg/ml) from GlycArt, Schlieren, Switzerland. Antibody buffer included histidine, trehalose and polysorbate 20.

Both solutions were diluted appropriately in PBS from stock for prior injections.

Cell lines and culture conditions

Z138 human B-Cell Non-Hodgkin-lymphoma (NHL) cells were originally obtained from Glycart (Mantle cell lymphoma-MCL). Tumor cell line was routinely cultured in DMEM medium (PAA, Laboratories, Austria) supplemented with 10% fetal bovine serum (PAA Laboratories, Austria) and 2 mM L-glutamine at 37°C in a water-saturated atmosphere at 5% $CO_2$. Passage 2 was used for transplantation.

Animals

Female SCID beige mice; age 4-5 weeks at arrival (purchased from Bomholtgard, Ry, Denmark) were maintained under specific-pathogen-free condition with daily cycles of 12 h light /12 h darkness according to committed guidelines (GV-Solas; Felasa; TierschG). Experimental study protocol was reviewed and approved by local government. After arrival animals were maintained in the quarantine part of the animal facility for one week to get accustomed to new environment and for observation. Continuous health monitoring was carried out on regular basis. Diet food (Provimi Kliba 3337) and water (acidified pH 2.5-3) were provided ad libitum.

Monitoring

Animals were controlled daily for clinical symptoms and detection of adverse effects. For monitoring throughout the experiment body weight of animals was documented two times weekly and tumor volume was measured by caliper beginning at staging.

Treatment of animals

Animal treatment started at day of randomisation, 14 days after s.c. cell transplantation. Humanized anti CD20 antibody (B-HH6-B-KV1 GE and wt) receiving groups and the corresponding vehicle group were treated i.v. q7d on study day 14, 20, 27 and 34 at the indicated dosage of 10 mg/kg.

Tumor growth inhibition study in vivo

Tumor bearing animals receiving vehicle control had to be excluded 19 days after treatment initiation due to tumor burden. Treatment of animals with weekly B-HH6-B-KV1 as wt or glycoengineered (B-HH6-B-KV1 GE and wt) at 10 mg/kg inhibited xenograft outgrowth shortly after start of treatment. At time of control termination all antibody tumors regressed and later most of Z138 tumor xenografts showed complete remission. No significant differences were observed between wt and glycoengineered versions of anti CD20 antibody B-HH6-B-KV1 in this xenograft model. This was not unlikely since mice do not express the correct Fc receptor on their NK cells and furthermore SCID beige mice are thought to be incompetent for NK-mediated ADCC due to severe triple immunodeficiency. Therefore s.c. xenografts models in SCID beige mice are not appropriate for mimicking human ADCC mediated effect with glycoengineered modified antibodies.

## Claims

1. A type II anti-CD20 antibody, **characterized in that**, at least 40% or more of the oligosaccharides of the Fc region of said type II anti-CD20 antibody are non-fucosylated,
for use in the treatment of CD20 expressing cancer
in combination with an a Bcl-2 protein binding inhibitor with an IC50 of the anti-Bcl-2 inhibitory activity of 5 μM or

less wherein the the IC50 of the inhibition of the Bcl-2 protein activity is determined in an homogenous time resolved fluorescence (HTRF) Assay.

2. The antibody according to claim 1 wherein the IC50 of anti-Bcl-2 inhibitory activity of the a Bcl-2 protein binding inhibitor is 1μM or less.

3. The antibody according to any one of claims 1 to 2, wherein said type II anti-CD20 antibody is a humanized B-Ly1 antibody.

4. The antibody according to any one of claims 1 to 3, wherein said type II anti-CD20 antibody has increased antibody dependent cellular cytotoxicity (ADCC).

5. The antibody according to any one of claims 1 to 4, wherein one or more additional other cytotoxic, chemotherapeutic or anti-cancer agents, or compounds that enhance the effects of such agents are administered.

6. The antibody according to any one of claims 1 to 10 wherein the CD20 expressing cancer is a B-Cell Non-Hodgkin's lymphoma (NHL).

7. A kit comprising a type II anti-CD20 antibody, **characterized in that**, at least 40% or more of the oligosaccharides of the Fc region of said type II anti-CD20 antibody are non-fucosylated, and an a Bcl-2 protein binding inhibitor with an IC50 of the anti-Bcl-2 inhibitory activity of 5 μM or less wherein the the IC50 of the inhibition of the Bcl-2 protein activity is determined in an homogenous time resolved fluorescence (HTRF) Assay, for the combination treatment of a patient suffering from a CD20 expressing cancer.

8. The Kit according to claim 7, wherein said type II anti-CD20 antibody is a humanized B-Ly1 antibody.

Fig. 1

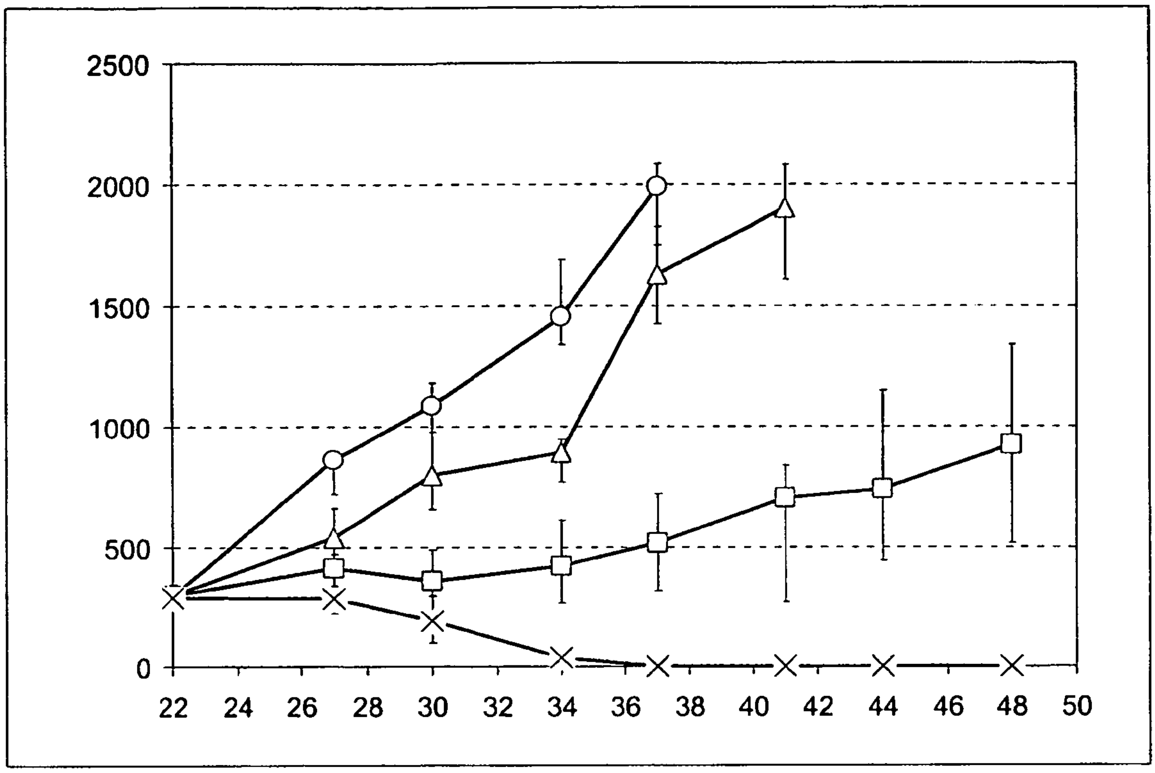
2500
2000
1500
1000
500
0
22
24
26
28
30
32
34
36
38
40
42
44
46
48
50

Fig. 2

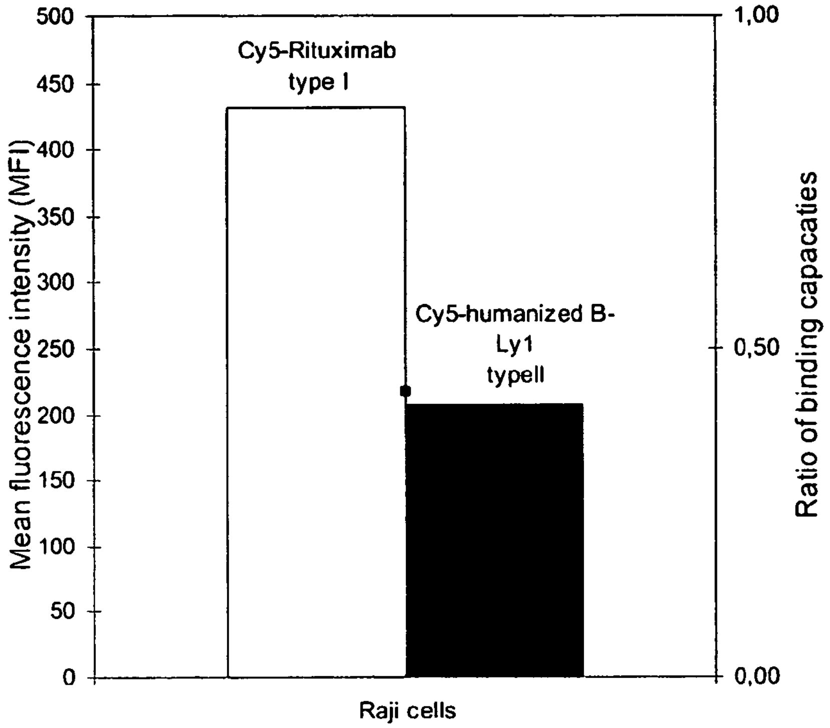
500
450
400
350
300
250
200
150
100
50
0
Mean fluorescence intensity (MFI)
Cy5-Rituximab
type I
Cy5-humanized B-
Ly1
typell
1,00
0,50
0,00
Ratio of binding capacaties
Raji cells

Fig. 3

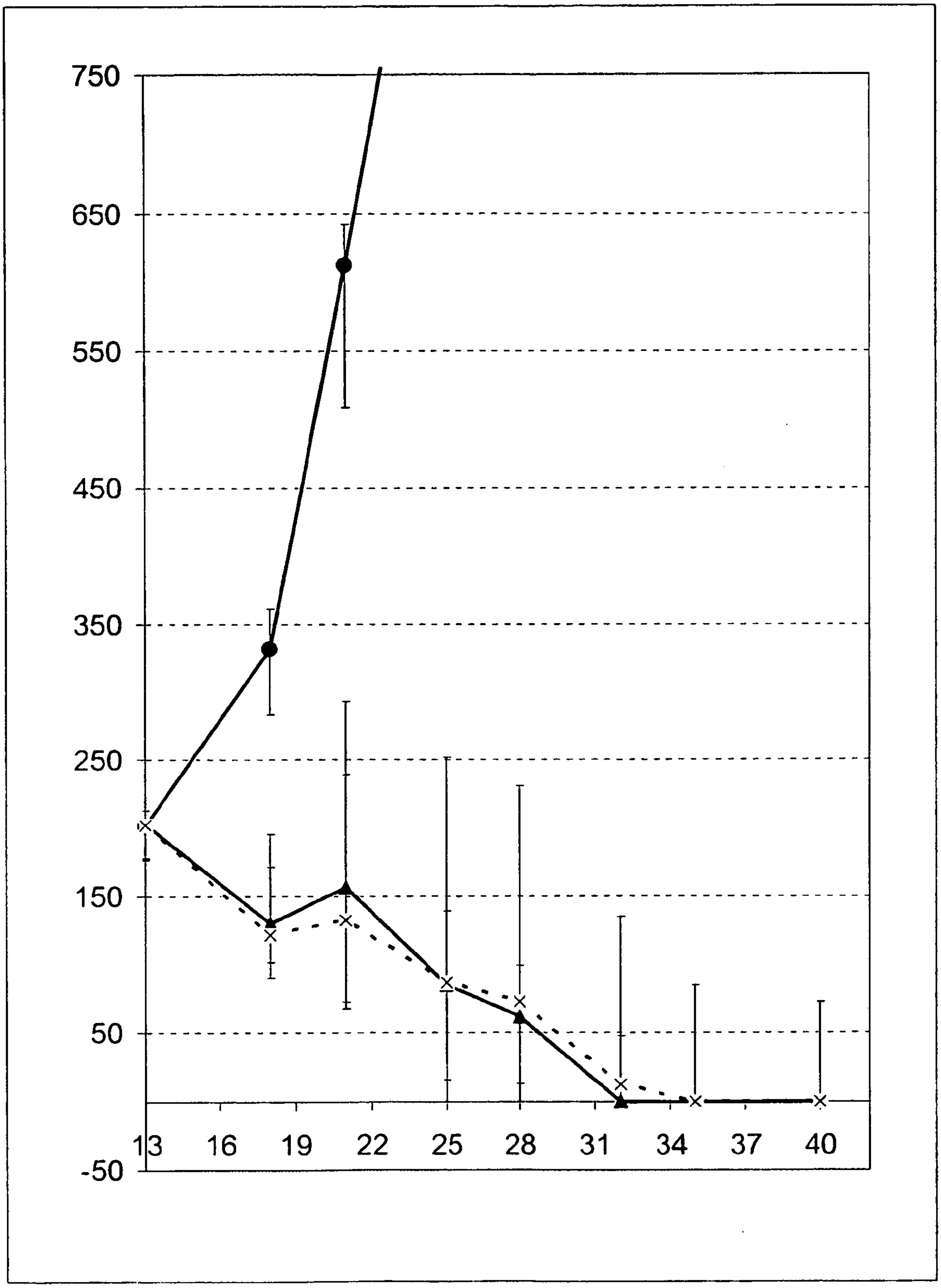

Europäisches Patentamt
European Patent Office
Office européen des brevets

# EUROPEAN SEARCH REPORT

Application Number
EP 13 15 5433

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| Y | SMITH MITCHELL R ET AL: "Enhanced efficacy of therapy with antisense BCL-2 oligonucleotides plus anti-CD20 monoclonal antibody in scid mouse/human lymphoma xenografts", MOLECULAR CANCER THERAPEUTICS, vol. 3, no. 12, December 2004 (2004-12), pages 1693-1699, XP002464965, ISSN: 1535-7163 * the whole document * | 1-8 | INV. A61K39/395 A61P35/00 |
| Y,D | WO 2005/044859 A (GLYCART BIOTECHNOLOGY AG [CH]; UMANA PABLO [CH]; BRUENKER PETER [CH];) 19 May 2005 (2005-05-19) * paragraphs [0040], [0041], [0114] - [0116], [0163], [0176] - [0178], [0238], [0240], [0241]; claims 1-259 * | 1-8 | |
| Y | UMANA PABLO ET AL: "Novel 3(rd) generation humanized type IICD20 antibody with glycoengineered fc and modified elbow hinge for enhanced ADCC and superior apoptosis induction.", BLOOD, vol. 108, no. 11, Part 1, November 2006 (2006-11), page 72A, XP002464966, & 48TH ANNUAL MEETING OF THE AMERICAN-SOCIETY-OF-HEMATOLOGY; ORLANDO, FL, USA; DECEMBER 09 -12, 2006 ISSN: 0006-4971 * the whole document * | 1-8 | TECHNICAL FIELDS SEARCHED (IPC): A61K |
| | -/-- | | |

The present search report has been drawn up for all claims

| Place of search | Date of completion of the search | Examiner |
|---|---|---|
| Munich | 8 May 2013 | Bayer, Annette |

1

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

Europäisches Patentamt
European Patent Office
Office européen des brevets

# EUROPEAN SEARCH REPORT

Application Number
EP 13 15 5433

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| A | GLENNIE MARTIN J ET AL: "Mechanisms of killing by anti-CD20 monoclonal antibodies", MOLECULAR IMMUNOLOGY, vol. 44, no. 16, September 2007 (2007-09), pages 3823-3837, XP002464967, ISSN: 0161-5890 * the whole document, especially page 3826, left-hand column, second paragarph; page 3831 ff item 4 and 5 * ----- | 1-8 | |
| A | SHAN D ET AL: "Synergistic effects of the fenretinide (4-HPR) and anti-CD20 monoclonal antibodies on apoptotis induction of malignant human B cells", CLINICAL CANCER RESEARCH, THE AMERICAN ASSOCIATION FOR CANCER RESEARCH, US, vol. 7, no. 8, August 2001 (2001-08), pages 2490-2495, XP003002434, ISSN: 1078-0432 * the whole document, especially figures 5 and 6 * ----- | 1-8 | TECHNICAL FIELDS SEARCHED (IPC) |

The present search report has been drawn up for all claims

1

| Place of search | Date of completion of the search | Examiner |
|---|---|---|
| Munich | 8 May 2013 | Bayer, Annette |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

## ANNEX TO THE EUROPEAN SEARCH REPORT ON EUROPEAN PATENT APPLICATION NO. EP 13 15 5433

This annex lists the patent family members relating to the patent documents cited in the above-mentioned European search report.
The members are as contained in the European Patent Office EDP file on
The European Patent Office is in no way liable for these particulars which are merely given for the purpose of information.

08-05-2013

| Patent document cited in search report | Publication date | Patent family member(s) | Publication date |
|---|---|---|---|
| WO 2005044859 A | 19-05-2005 | AT 463513 T | 15-04-2010 |
| | | AU 2004287643 A1 | 19-05-2005 |
| | | BR PI0416262 A | 09-01-2007 |
| | | CA 2544865 A1 | 19-05-2005 |
| | | CN 1902231 A | 24-01-2007 |
| | | CN 102373214 A | 14-03-2012 |
| | | CN 102373215 A | 14-03-2012 |
| | | DK 1692182 T3 | 31-05-2010 |
| | | EA 200600905 A1 | 29-12-2006 |
| | | EA 201100128 A1 | 30-06-2011 |
| | | EC SP066603 A | 17-10-2006 |
| | | EP 1692182 A2 | 23-08-2006 |
| | | EP 2077282 A2 | 08-07-2009 |
| | | EP 2348051 A2 | 27-07-2011 |
| | | EP 2380910 A1 | 26-10-2011 |
| | | EP 2380911 A1 | 26-10-2011 |
| | | ES 2341009 T3 | 14-06-2010 |
| | | HR P20100303 T1 | 31-07-2010 |
| | | JP 4653109 B2 | 16-03-2011 |
| | | JP 2008500017 A | 10-01-2008 |
| | | JP 2010081940 A | 15-04-2010 |
| | | JP 2012254083 A | 27-12-2012 |
| | | KR 20060130579 A | 19-12-2006 |
| | | KR 20110129990 A | 02-12-2011 |
| | | KR 20120104419 A | 20-09-2012 |
| | | MA 31040 B1 | 04-01-2010 |
| | | MX PA06004836 A | 06-07-2006 |
| | | NZ 547589 A | 31-05-2009 |
| | | NZ 588860 A | 30-03-2012 |
| | | PT 1692182 E | 13-05-2010 |
| | | RS 51334 B | 28-02-2011 |
| | | SG 160348 A1 | 29-04-2010 |
| | | SI 1692182 T1 | 30-06-2010 |
| | | US 2005123546 A1 | 09-06-2005 |
| | | US 2009010921 A1 | 08-01-2009 |
| | | WO 2005044859 A2 | 19-05-2005 |
| | | ZA 200604547 A | 25-07-2007 |

EPO FORM P0459

For more details about this annex : see Official Journal of the European Patent Office, No. 12/82

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- US 5202238 A
- US 5204244 A
- WO 2005044859 A
- WO 2004035607 A
- WO 2005103081 A
- WO 2004056312 A
- US 6602684 B
- US 5736137 A, Andersen, K.C.
- WO 2007031875 A
- WO 2004065540 A
- WO 99154342 A
- WO 9508350 A
- WO 1999051259 A
- WO 2002017852 A
- WO 2004056971 A
- US 5734033 A
- WO 2006099667 A
- US 2007027135 A
- WO 371191 A
- WO 2004106328 A
- WO 2006089397 A
- WO 9960023 A
- US 3773919 A


### Non-patent literature cited in the description


- **VALENTINE, M.A. et al.** *J. Biol. Chem.,* 1989, vol. 264 (19), 11282-11287
- **EINFIELD, D.A. et al.** *EMBO J.,* 1988, vol. 7 (3), 711-717
- **ANDERSON, K.C. et al.** *Blood,* 1984, vol. 63 (6), 1424-1433
- **TEDDER, T.F. et al.** *J, Immunol.,* 1985, vol. 135 (2), 973-979
- **KOMAROMY, M. et al.** *NAR,* 1983, vol. 11, 6775-6785
- **TEDDER, T.F. et al.** *Eur. J. Immunol.,* 1986, vol. 16, 881-887
- **TEDDER, T.F. et al.** *J. Cell. Biochem.,* 1990, vol. 14D, 195
- **CRAGG, M.S. et al.** *Blood,* 2004, vol. 103, 2738-2743
- **CRAGG, M.S. et al.** *Blood,* 2003, vol. 101, 1045-1052
- **CORY. S. ; ADAMS, J.M.** *Nature Reviews Cancer,* 2002, vol. 2, 647-656
- **ADAMS.** *Genes und Development,* 2003, vol. 17, 2481-2495
- **DANIAL, N.N. ; KORSMEYER, S.J.** *Cell,* 2004, vol. 116, 205-219
- **HUANG ; STRASSER.** *Ce11,* 2000, vol. 103, 839-842
- **CHENG et al.** *Molecular Cell,* 2001, vol. 8, 705-711
- **WEI, M.C. et al.** *Science,* 2001, vol. 292, 727-730
- **ZONG, W.X. et al.** *Genes and Development,* 2001, vol. 15 (148), 1-1486
- **WANG, K.** *Genes and Development,* 2001, vol. 15, 2922-2933
- **GREEN, D.R. ; KROEMER, G.** *Science,* 2004, vol. 305, 626-629
- **SATTLER et al.** *Science,* 1997, vol. 275, 983-986
- **HSU, Y.T. et al.** *Journal of Biological Chemistry,* 1997, vol. 272, 13289-1 3834
- **WOLTER, K.G. et al.** *Journal of Cell Biology,* 1997, vol. 139, 1281-92
- **NECHUSHTAN, A. et al.** *EMBO Journal,* 1999, vol. 18, 2330-2341
- **SUZUKI et al.** *Cell,* 2000, vol. 103, 645-654
- **SCHINZEL, A. et al.** *J Cell Biol,* 2004, vol. 164, 1021-1032
- **LUCKEN-ARDJOMANDE, S. ; MARTINOU, J.C.** *J Cell Sci,* 2005, vol. 118, 473-483
- **WEI, M.C. et al.** *Genes und Development,* 2000, vol. 14, 2060-2071
- **KUWANA, T. et al.** *Cell,* 2002, vol. 111, 331-342
- **ROUCOU, X. et al.** *Biochemical Journal,* 2002, vol. 368, 915-921
- **CARTRON, P.F. et al.** *Mol Cell,* 2004, vol. 16, 807-818
- **OLTVAI, Z.N. et al.** *Cell,* 1993, vol. 74, 609-619
- **ZHA, H. ; REED, J.** *Journal of Biological Chemistry,* 1997, vol. 272, 31482-88
- **WANG, K. et al.** *Molecular und Cellular Biology,* 1998, vol. 18, 6083-6089
- **MIKHAILOV, V. et al.** *Journal of Biological Chemistry,* 2001, vol. 276, 18361-18374

- **LINDSTEN, T. et al.** *Molecular Cell,* 2000, vol. 6, 1389-1399
- **ZONG, W.X. et al.** *Journal of Ce11 Biology,* 2003, vol. 162, 59-69
- **HSU, Y.T. et al.** *PNAS,* 1997, vol. 94, 3668-3672
- **ANTONSSON, B. et al.** *Journal of Biological Chemistry,* 2001, vol. 276, 11615-11623
- **NECHUSHTAN, A. et al.** *Journal of Cell Biology,* 2001, vol. 153, 1265-1276
- **MIKHAILOV, V. et al.** *Journal of Biological Chemistry,* 2003, vol. 278, 5367-5376
- **ZHAI, D. et al.** *Cell Death and Differentiation,* 2006, vol. 13, 1419-1421
- **SMITH, M. R. et al.** *Molecular Cancer Therapeutics,* 2004, vol. 3 (12), 1693-1699
- **RAMANARAYANAN, J. et al.** *British Journal of Haematology,* 2004, vol. 127 (5), 519-530
- **MORRISON, S.L. et al.** *Proc. Natl. Acad Sci. USA,* 1984, vol. 81, 6851-6855
- **RIECHMANN, L. et al.** *Nature,* 1988, vol. 332, 323-327
- **NEUBERGER, M.S. et al.** *Nature,* 1985, vol. 314, 268-270
- **VAN DIJK, M.A. ; VAN DE WINKEL, J.G.** *Curr. Opin. Pharmacol.,* 2001, vol. 5, 368-374
- **KELLERMANN, S. A. et al.** *Curr Opin Biotechnol.,* 2002, vol. 13, 593-597
- **KABAT et al.** Sequences of Proteins of Immunological Interest. Public Health Service, 1991
- **PRESS et al.** *Blood,* 1987, vol. 69/2, 584-591
- **UMANA, P. et al.** *Nature Biotechnol.,* 1999, vol. 17, 176-180
- **REFF.** *Blood,* 1994, vol. 83 (2), 435-445
- **POPPEMA, S. ; VISSER, L.** *Biotest Bulletin,* 1987, vol. 3, 131-139
- **JENKINS, N. et al.** *Nature Biotechnol.,* 1996, vol. 14, 975-981
- **CUMMING, D.A. et al.** *Glycobiology,* 1991, vol. 1, 115-130
- **WRIGHT, A. ; MONISON, S. L.** *Trends Biotech.,* 1997, vol. 15, 26-32
- **WRIGHT, A. ; MORRISON, S. L.** *Trends Biotech.,* 1997, vol. 15, 26-32
- **LIFELY, M. R. et al.** *Glycobiology,* 1995, vol. 5, 813-822
- **JEFFERIS, R. et al.** *Immunol. Rev.,* 1998, vol. 163, 59-76
- **WRIGHT, A. ; MORRISON, S. L.** *Trends Biotechnol.,* 1997, vol. 15, 26-32
- **CORY, S. ; ADAMS, J.M.** *Nature Reviews Cancer,* 2002, vol. 2, 647-656
- **PETROS, A. M.** *Biochim Biophys Acta,* 2004, vol. 1644, 83-94
- **HONDA, T. ; ROUNDS BV BORE, L. et al.** *J Med Chem.,* 2000, vol. 43, 4233-4246
- **KONOPLEVA, M. et al.** *Blood,* 2002, vol. 99, 326-35
- **COREY, S. et al.** *Cancer Cell,* 2005, vol. 8, 5-6
- **SHOEMAKER, A.R. et al.** *Cancer Research,* 2006, vol. 66, 8731-8739
- **WALENSKY, L.D.** *Cell Death and Differentiation,* 2006, vol. 13, 1339-1350
- Remington's Pharmaceutical Sciences. 1980
- **CHANG, H. et al.** *Leuk. Lymphoma,* 1992, vol. 8, 129-136

SEQUENCE LISTING

<110> F. Hoffmann-La Roche AG

<120> Combination therapy of a type II anti-CD20 antibody with an anti-Bcl-2 active agent

<130> 24555

<150> EP 07020120
<151> 2007-10-15

<160> 20

<170> PatentIn version 3.2

<210> 1
<211> 112
<212> PRT
<213> Mus sp.

<220>
<221> MISC_FEATURE
<223> amino acid sequence of variable region of the heavy chain (VH) of murine monoclonal anti-CD20 antibody B-Ly1

<400> 1

```
Gly Pro Glu Leu Val Lys Pro Gly Ala Ser Val Lys Ile Ser Cys Lys
1               5                   10                  15

Ala Ser Gly Tyr Ala Phe Ser Tyr Ser Trp Met Asn Trp Val Lys Leu
            20                  25                  30

Arg Pro Gly Gln Gly Leu Glu Trp Ile Gly Arg Ile Phe Pro Gly Asp
        35                  40                  45

Gly Asp Thr Asp Tyr Asn Gly Lys Phe Lys Gly Lys Ala Thr Leu Thr
    50                  55                  60

Ala Asp Lys Ser Ser Asn Thr Ala Tyr Met Gln Leu Thr Ser Leu Thr
65                  70                  75                  80

Ser Val Asp Ser Ala Val Tyr Leu Cys Ala Arg Asn Val Phe Asp Gly
                85                  90                  95

Tyr Trp Leu Val Tyr Trp Gly Gln Gly Thr Leu Val Thr Val Ser Ala
```

100 105 110

<210> 2
<211> 103
<212> PRT
<213> Mus sp.

<220>
<221> MISC_FEATURE
<223> amino acid sequence of variable region of the light chain (VL) of murine monoclonal anti-CD20 antibody B-Ly1

<400> 2

```
Asn Pro Val Thr Leu Gly Thr Ser Ala Ser Ile Ser Cys Arg Ser Ser
1               5                   10                  15

Lys Ser Leu Leu His Ser Asn Gly Ile Thr Tyr Leu Tyr Trp Tyr Leu
            20                  25                  30

Gln Lys Pro Gly Gln Ser Pro Gln Leu Leu Ile Tyr Gln Met Ser Asn
        35                  40                  45

Leu Val Ser Gly Val Pro Asp Arg Phe Ser Ser Ser Gly Ser Gly Thr
    50                  55                  60

Asp Phe Thr Leu Arg Ile Ser Arg Val Glu Ala Glu Asp Val Gly Val
65                  70                  75                  80

Tyr Tyr Cys Ala Gln Asn Leu Glu Leu Pro Tyr Thr Phe Gly Gly Gly
                85                  90                  95

Thr Lys Leu Glu Ile Lys Arg
            100
```

<210> 3
<211> 119
<212> PRT
<213> Artificial

<220>
<223> amino acid sequences of variable region of the heavy chain (VH) of humanized B-Ly1 antibody (B-HH2)

<400> 3

Gln Val Gln Leu Val Gln Ser Gly Ala Glu Val Lys Lys Pro Gly Ser
1 5 10 15

Ser Val Lys Val Ser Cys Lys Ala Ser Gly Tyr Ala Phe Ser Tyr Ser
20 25 30

Trp Met Asn Trp Val Arg Gln Ala Pro Gly Gln Gly Leu Glu Trp Met
35 40 45

Gly Arg Ile Phe Pro Gly Asp Gly Asp Thr Asp Tyr Asn Gly Lys Phe
50 55 60

Lys Gly Arg Val Thr Ile Thr Ala Asp Lys Ser Thr Ser Thr Ala Tyr
65 70 75 80

Met Glu Leu Ser Ser Leu Arg Ser Glu Asp Thr Ala Val Tyr Tyr Cys
85 90 95

Ala Arg Asn Val Phe Asp Gly Tyr Trp Leu Val Tyr Trp Gly Gln Gly
100 105 110

Thr Leu Val Thr Val Ser Ser
115

<210> 4
<211> 119
<212> PRT
<213> Artificial

<220>
<223> amino acid sequences of variable region of the heavy chain (VH) of humanized B-Ly1 antibody (B-HH3)

<400> 4

Gln Val Gln Leu Val Gln Ser Gly Ala Glu Val Lys Lys Pro Gly Ser
1 5 10 15

Ser Val Lys Val Ser Cys Lys Ala Ser Gly Tyr Ala Phe Ser Tyr Ser
20 25 30

Trp Met Asn Trp Val Arg Gln Ala Pro Gly Gln Gly Leu Glu Trp Met
35 40 45

Gly Arg Ile Phe Pro Gly Asp Gly Asp Thr Asp Tyr Asn Gly Lys Phe
50 55 60

Lys Gly Arg Val Thr Ile Thr Ala Asp Lys Ser Thr Ser Thr Ala Tyr
65 70 75 80

Met Glu Leu Ser Ser Leu Arg Ser Glu Asp Thr Ala Val Tyr Leu Cys
85 90 95

Ala Arg Asn Val Phe Asp Gly Tyr Trp Leu Val Tyr Trp Gly Gln Gly
100 105 110

Thr Leu Val Thr Val Ser Ser
115

<210> 5
<211> 119
<212> PRT
<213> Artificial

<220>
<223> amino acid sequences of variable region of the heavy chain (VH) of humanized B-Ly1 antibody (B-HH4)

<400> 5

Gln Val Gln Leu Val Gln Ser Gly Ala Glu Val Lys Lys Pro Gly Ala
1 5 10 15

Ser Val Lys Val Ser Cys Lys Val Ser Gly Tyr Ala Phe Ser Tyr Ser
20 25 30

Trp Met Asn Trp Val Arg Gln Ala Pro Gly Gln Gly Leu Glu Trp Met
35 40 45

Gly Arg Ile Phe Pro Gly Asp Gly Asp Thr Asp Tyr Asn Gly Lys Phe
50 55 60

Lys Gly Arg Val Thr Ile Thr Ala Asp Lys Ser Thr Ser Thr Ala Tyr
65 70 75 80

Met Glu Leu Ser Ser Leu Arg Ser Glu Asp Thr Ala Val Tyr Tyr Cys
85 90 95

Ala Arg Asn Val Phe Asp Gly Tyr Trp Leu Val Tyr Trp Gly Gln Gly
100 105 110

Thr Leu Val Thr Val Ser Ser
115

<210> 6
<211> 119
<212> PRT
<213> Artificial

<220>
<223> amino acid sequences of variable region of the heavy chain (VH) of humanized B-Ly1 antibody (B-HH5)

<400> 6

Gln Val Gln Leu Val Gln Ser Gly Ala Glu Val Lys Lys Pro Gly Ser
1 5 10 15

Ser Val Lys Val Ser Cys Lys Ala Ser Gly Tyr Ala Phe Ser Tyr Ser
20 25 30

Trp Met Ser Trp Val Arg Gln Ala Pro Gly Gln Gly Leu Glu Trp Met
35 40 45

Gly Arg Ile Phe Pro Gly Asp Gly Asp Thr Asp Tyr Asn Gly Lys Phe
50 55 60

Lys Gly Arg Val Thr Ile Thr Ala Asp Lys Ser Thr Ser Thr Ala Tyr
65 70 75 80

Met Glu Leu Ser Ser Leu Arg Ser Glu Asp Thr Ala Val Tyr Tyr Cys
85 90 95

Ala Arg Asn Val Phe Asp Gly Tyr Trp Leu Val Tyr Trp Gly Gln Gly
100 105 110

Thr Leu Val Thr Val Ser Ser
115

<210> 7
<211> 119
<212> PRT
<213> Artificial

<220>
<223> amino acid sequences of variable region of the heavy chain (VH) of humanized B-Ly1 antibody (B-HH6)

<400> 7

Gln Val Gln Leu Val Gln Ser Gly Ala Glu Val Lys Lys Pro Gly Ser
1 5 10 15

Ser Val Lys Val Ser Cys Lys Ala Ser Gly Tyr Ala Phe Ser Tyr Ser
20 25 30

Trp Ile Asn Trp Val Arg Gln Ala Pro Gly Gln Gly Leu Glu Trp Met
35 40 45

Gly Arg Ile Phe Pro Gly Asp Gly Asp Thr Asp Tyr Asn Gly Lys Phe
50 55 60

Lys Gly Arg Val Thr Ile Thr Ala Asp Lys Ser Thr Ser Thr Ala Tyr
65 70 75 80

Met Glu Leu Ser Ser Leu Arg Ser Glu Asp Thr Ala Val Tyr Tyr Cys
85 90 95

Ala Arg Asn Val Phe Asp Gly Tyr Trp Leu Val Tyr Trp Gly Gln Gly
100 105 110

Thr Leu Val Thr Val Ser Ser
115

<210> 8
<211> 119
<212> PRT
<213> Artificial

<220>
<223> amino acid sequences of variable region of the heavy chain (VH) of humanized B-Ly1 antibody (B-HH7)

<400> 8

Gln Val Gln Leu Val Gln Ser Gly Ala Glu Val Lys Lys Pro Gly Ser
1 5 10 15

Ser Val Lys Val Ser Cys Lys Ala Ser Gly Tyr Ala Phe Ser Tyr Ser

20 25 30

Trp Ile Ser Trp Val Arg Gln Ala Pro Gly Gln Gly Leu Glu Trp Met
35 40 45

Gly Arg Ile Phe Pro Gly Asp Gly Asp Thr Asp Tyr Asn Gly Lys Phe
50 55 60

Lys Gly Arg Val Thr Ile Thr Ala Asp Lys Ser Thr Ser Thr Ala Tyr
65 70 75 80

Met Glu Leu Ser Ser Leu Arg Ser Glu Asp Thr Ala Val Tyr Tyr Cys
85 90 95

Ala Arg Asn Val Phe Asp Gly Tyr Trp Leu Val Tyr Trp Gly Gln Gly
100 105 110

Thr Leu Val Thr Val Ser Ser
115

<210> 9
<211> 119
<212> PRT
<213> Artificial

<220>
<223> amino acid sequences of variable region of the heavy chain (VH) of humanized B-Ly1 antibody (B-HH8)

<400> 9

Gln Val Gln Leu Val Gln Ser Gly Ala Glu Val Lys Lys Pro Gly Ala
1 5 10 15

Ser Val Lys Val Ser Cys Lys Ala Ser Gly Tyr Thr Phe Thr Tyr Ser
20 25 30

Trp Met Asn Trp Val Arg Gln Ala Pro Gly Gln Gly Leu Glu Trp Met
35 40 45

Gly Arg Ile Phe Pro Gly Asp Gly Asp Thr Asp Tyr Asn Gly Lys Phe
50 55 60

Lys Gly Arg Val Thr Ile Thr Ala Asp Lys Ser Thr Ser Thr Ala Tyr
65 70 75 80

Met Glu Leu Ser Ser Leu Arg Ser Glu Asp Thr Ala Val Tyr Tyr Cys
85 90 95

Ala Arg Asn Val Phe Asp Gly Tyr Trp Leu Val Tyr Trp Gly Gln Gly
100 105 110

Thr Leu Val Thr Val Ser Ser
115

<210> 10
<211> 119
<212> PRT
<213> Artificial

<220>
<223> amino acid sequences of variable region of the heavy chain (VH) of humanized B-Ly1 antibody (B-HH9)

<400> 10

Gln Val Gln Leu Val Gln Ser Gly Ala Glu Val Lys Lys Pro Gly Ala
1 5 10 15

Ser Val Lys Val Ser Cys Lys Ala Ser Gly Tyr Thr Phe Ser Tyr Ser
20 25 30

Trp Met Asn Trp Val Arg Gln Ala Pro Gly Gln Gly Leu Glu Trp Met
35 40 45

Gly Arg Ile Phe Pro Gly Asp Gly Asp Thr Asp Tyr Asn Gly Lys Phe
50 55 60

Lys Gly Arg Val Thr Ile Thr Ala Asp Lys Ser Thr Ser Thr Ala Tyr
65 70 75 80

Met Glu Leu Ser Ser Leu Arg Ser Glu Asp Thr Ala Val Tyr Tyr Cys
85 90 95

Ala Arg Asn Val Phe Asp Gly Tyr Trp Leu Val Tyr Trp Gly Gln Gly
100 105 110

Thr Leu Val Thr Val Ser Ser
115

<210> 11
<211> 119
<212> PRT
<213> Artificial

<220>
<223> amino acid sequences of variable region of the heavy chain (VH) of humanized B-Ly1 antibody (B-HL8)

<400> 11

Glu Val Gln Leu Val Glu Ser Gly Gly Gly Leu Val Lys Pro Gly Gly
1 5 10 15

Ser Leu Arg Leu Ser Cys Ala Ala Ser Gly Phe Thr Phe Ser Tyr Ser
20 25 30

Trp Met Asn Trp Val Arg Gln Ala Pro Gly Lys Gly Leu Glu Trp Val
35 40 45

Gly Arg Ile Phe Pro Gly Asp Gly Asp Thr Asp Tyr Asn Gly Lys Phe
50 55 60

Lys Gly Arg Val Thr Ile Thr Ala Asp Lys Ser Thr Ser Thr Ala Tyr
65 70 75 80

Met Glu Leu Ser Ser Leu Arg Ser Glu Asp Thr Ala Val Tyr Tyr Cys
85 90 95

Ala Arg Asn Val Phe Asp Gly Tyr Trp Leu Val Tyr Trp Gly Gln Gly
100 105 110

Thr Leu Val Thr Val Ser Ser
115

<210> 12
<211> 119
<212> PRT
<213> Artificial

<220>
<223> amino acid sequences of variable region of the heavy chain (VH) of humanized B-Ly1 antibody (B-HL10)

<400> 12

Glu Val Gln Leu Val Glu Ser Gly Gly Gly Leu Val Lys Pro Gly Gly
1 5 10 15

Ser Leu Arg Leu Ser Cys Ala Ala Ser Gly Phe Ala Phe Ser Tyr Ser
20 25 30

Trp Met Asn Trp Val Arg Gln Ala Pro Gly Lys Gly Leu Glu Trp Val
35 40 45

Gly Arg Ile Phe Pro Gly Asp Gly Asp Thr Asp Tyr Asn Gly Lys Phe
50 55 60

Lys Gly Arg Val Thr Ile Thr Ala Asp Lys Ser Thr Ser Thr Ala Tyr
65 70 75 80

Met Glu Leu Ser Ser Leu Arg Ser Glu Asp Thr Ala Val Tyr Tyr Cys
85 90 95

Ala Arg Asn Val Phe Asp Gly Tyr Trp Leu Val Tyr Trp Gly Gln Gly
100 105 110

Thr Leu Val Thr Val Ser Ser
115

<210> 13
<211> 119
<212> PRT
<213> Artificial

<220>
<223> amino acid sequences of variable region of the heavy chain (VH) of humanized B-Ly1 antibody (B-HL11)

<400> 13

Gln Val Gln Leu Val Glu Ser Gly Gly Gly Leu Val Lys Pro Gly Gly
1 5 10 15

Ser Leu Arg Leu Ser Cys Ala Ala Ser Gly Phe Thr Phe Ser Tyr Ser
20 25 30

Trp Met Asn Trp Val Arg Gln Ala Pro Gly Lys Gly Leu Glu Trp Val
35 40 45

Gly Arg Ile Phe Pro Gly Asp Gly Asp Thr Asp Tyr Asn Gly Lys Phe
50 55 60

Lys Gly Arg Val Thr Ile Thr Ala Asp Lys Ser Thr Ser Thr Ala Tyr
65 70 75 80

Met Glu Leu Ser Ser Leu Arg Ser Glu Asp Thr Ala Val Tyr Tyr Cys
85 90 95

Ala Arg Asn Val Phe Asp Gly Tyr Trp Leu Val Tyr Trp Gly Gln Gly
100 105 110

Thr Leu Val Thr Val Ser Ser
115

<210> 14
<211> 119
<212> PRT
<213> Artificial

<220>
<223> amino acid sequences of variable region of the heavy chain (VH) of humanized B-Ly1 antibody (B-HL12)

<400> 14

Glu Val Gln Leu Val Glu Ser Gly Ala Gly Leu Val Lys Pro Gly Gly
1 5 10 15

Ser Leu Arg Leu Ser Cys Ala Ala Ser Gly Phe Thr Phe Ser Tyr Ser
20 25 30

Trp Met Asn Trp Val Arg Gln Ala Pro Gly Lys Gly Leu Glu Trp Met
35 40 45

Gly Arg Ile Phe Pro Gly Asp Gly Asp Thr Asp Tyr Asn Gly Lys Phe
50 55 60

Lys Gly Arg Val Thr Ile Thr Ala Asp Lys Ser Thr Ser Thr Ala Tyr
65 70 75 80

Met Glu Leu Ser Ser Leu Arg Ser Glu Asp Thr Ala Val Tyr Tyr Cys
85 90 95

Ala Arg Asn Val Phe Asp Gly Tyr Trp Leu Val Tyr Trp Gly Gln Gly
100 105 110

Thr Leu Val Thr Val Ser Ser
115

<210> 15
<211> 119
<212> PRT
<213> Artificial

<220>
<223> amino acid sequences of variable region of the heavy chain (VH) of humanized B-Ly1 antibody (B-HL13)

<400> 15

Glu Val Gln Leu Val Glu Ser Gly Gly Gly Val Val Lys Pro Gly Gly
1 5 10 15

Ser Leu Arg Leu Ser Cys Ala Ala Ser Gly Phe Thr Phe Ser Tyr Ser
20 25 30

Trp Met Asn Trp Val Arg Gln Ala Pro Gly Lys Gly Leu Glu Trp Met
35 40 45

Gly Arg Ile Phe Pro Gly Asp Gly Asp Thr Asp Tyr Asn Gly Lys Phe
50 55 60

Lys Gly Arg Val Thr Ile Thr Ala Asp Lys Ser Thr Ser Thr Ala Tyr
65 70 75 80

Met Glu Leu Ser Ser Leu Arg Ser Glu Asp Thr Ala Val Tyr Tyr Cys
85 90 95

Ala Arg Asn Val Phe Asp Gly Tyr Trp Leu Val Tyr Trp Gly Gln Gly
100 105 110

Thr Leu Val Thr Val Ser Ser
115

<210> 16
<211> 119
<212> PRT
<213> Artificial

<220>
<223> amino acid sequences of variable region of the heavy chain (VH) of humanized B-Ly1 antibody (B-HL14)

<400> 16

Glu Val Gln Leu Val Glu Ser Gly Gly Gly Leu Lys Lys Pro Gly Gly
1 5 10 15

Ser Leu Arg Leu Ser Cys Ala Ala Ser Gly Phe Thr Phe Ser Tyr Ser
20 25 30

Trp Met Asn Trp Val Arg Gln Ala Pro Gly Lys Gly Leu Glu Trp Met
35 40 45

Gly Arg Ile Phe Pro Gly Asp Gly Asp Thr Asp Tyr Asn Gly Lys Phe
50 55 60

Lys Gly Arg Val Thr Ile Thr Ala Asp Lys Ser Thr Ser Thr Ala Tyr
65 70 75 80

Met Glu Leu Ser Ser Leu Arg Ser Glu Asp Thr Ala Val Tyr Tyr Cys
85 90 95

Ala Arg Asn Val Phe Asp Gly Tyr Trp Leu Val Tyr Trp Gly Gln Gly
100 105 110

Thr Leu Val Thr Val Ser Ser
115

<210> 17
<211> 119
<212> PRT
<213> Artificial

<220>
<223> amino acid sequences of variable region of the heavy chain (VH) of humanized B-Ly1 antibody (B-HL15)

<400> 17

Glu Val Gln Leu Val Glu Ser Gly Gly Gly Leu Val Lys Pro Gly Ser

1 5 10 15

Ser Leu Arg Leu Ser Cys Ala Ala Ser Gly Phe Thr Phe Ser Tyr Ser
20 25 30

Trp Met Asn Trp Val Arg Gln Ala Pro Gly Lys Gly Leu Glu Trp Met
35 40 45

Gly Arg Ile Phe Pro Gly Asp Gly Asp Thr Asp Tyr Asn Gly Lys Phe
50 55 60

Lys Gly Arg Val Thr Ile Thr Ala Asp Lys Ser Thr Ser Thr Ala Tyr
65 70 75 80

Met Glu Leu Ser Ser Leu Arg Ser Glu Asp Thr Ala Val Tyr Tyr Cys
85 90 95

Ala Arg Asn Val Phe Asp Gly Tyr Trp Leu Val Tyr Trp Gly Gln Gly
100 105 110

Thr Leu Val Thr Val Ser Ser
115

<210> 18
<211> 119
<212> PRT
<213> Artificial

<220>
<223> amino acid sequences of variable region of the heavy chain (VH) of humanized B-Ly1 antibody (B-HL16)

<400> 18

Glu Val Gln Leu Val Glu Ser Gly Gly Gly Leu Val Lys Pro Gly Gly
1 5 10 15

Ser Leu Arg Val Ser Cys Ala Ala Ser Gly Phe Thr Phe Ser Tyr Ser
20 25 30

Trp Met Asn Trp Val Arg Gln Ala Pro Gly Lys Gly Leu Glu Trp Met
35 40 45

Gly Arg Ile Phe Pro Gly Asp Gly Asp Thr Asp Tyr Asn Gly Lys Phe
50 55 60

Lys Gly Arg Val Thr Ile Thr Ala Asp Lys Ser Thr Ser Thr Ala Tyr
65 70 75 80

Met Glu Leu Ser Ser Leu Arg Ser Glu Asp Thr Ala Val Tyr Tyr Cys
85 90 95

Ala Arg Asn Val Phe Asp Gly Tyr Trp Leu Val Tyr Trp Gly Gln Gly
100 105 110

Thr Leu Val Thr Val Ser Ser
115

<210> 19
<211> 119
<212> PRT
<213> Artificial

<220>
<223> amino acid sequences of variable region of the heavy chain (VH) of humanized B-Ly1 antibody (B-HL17)

<400> 19

Glu Val Gln Leu Val Glu Ser Gly Gly Gly Leu Val Lys Pro Gly Gly
1 5 10 15

Ser Leu Arg Leu Ser Cys Ala Ala Ser Gly Phe Thr Phe Ser Tyr Ser
20 25 30

Trp Met Asn Trp Val Arg Gln Ala Pro Gly Lys Gly Leu Glu Trp Met
35 40 45

Gly Arg Ile Phe Pro Gly Asp Gly Asp Thr Asp Tyr Asn Gly Lys Phe
50 55 60

Lys Gly Arg Val Thr Ile Thr Ala Asp Lys Ser Thr Ser Thr Ala Tyr
65 70 75 80

Met Glu Leu Ser Ser Leu Arg Ser Glu Asp Thr Ala Val Tyr Tyr Cys
85 90 95

```
Ala Arg Asn Val Phe Asp Gly Tyr Trp Leu Val Tyr Trp Gly Gln Gly
            100                 105                 110

Thr Leu Val Thr Val Ser Ser
        115
```

<210> 20
<211> 115
<212> PRT
<213> Artificial

<220>
<223> amino acid sequences of variable region of the light chain (VL) of humanized B-Ly1 antibody B-KV1

<400> 20

```
Asp Ile Val Met Thr Gln Thr Pro Leu Ser Leu Pro Val Thr Pro Gly
1               5                   10                  15

Glu Pro Ala Ser Ile Ser Cys Arg Ser Ser Lys Ser Leu Leu His Ser
            20                  25                  30

Asn Gly Ile Thr Tyr Leu Tyr Trp Tyr Leu Gln Lys Pro Gly Gln Ser
        35                  40                  45

Pro Gln Leu Leu Ile Tyr Gln Met Ser Asn Leu Val Ser Gly Val Pro
    50                  55                  60

Asp Arg Phe Ser Gly Ser Gly Ser Gly Thr Asp Phe Thr Leu Lys Ile
65                  70                  75                  80

Ser Arg Val Glu Ala Glu Asp Val Gly Val Tyr Tyr Cys Ala Gln Asn
                85                  90                  95

Leu Glu Leu Pro Tyr Thr Phe Gly Gly Gly Thr Lys Val Glu Ile Lys
            100                 105                 110

Arg Thr Val
        115
```